# EUROPEAN PATENT SPECIFICATION

(11) **EP 1 846 422 B1**
(45) Date of publication and mention of the grant of the patent: **13.04.2016**
(21) Application number: 06715903.8
(22) Date of filing: 07.02.2006
(51) Int. Cl.: C07F 1/10

(54) **ORGANIC SILVER COMPLEXES, THEIR PREPARATION METHODS AND THEIR METHODS FOR FORMING THIN LAYERS**
ORGANISCHE SILBERKOMPLEXE, DEREN HERSTELLUNGSVERFAHREN UND DEREN VERFAHREN ZUR BILDUNG VON DÜNNEN SCHICHTEN
COMPLEXES CONTENANT DE L'ARGENT ORGANIQUE, PROCEDES DE PREPARATION ASSOCIES ET PROCEDES POUR FORMER DES COUCHES MINCES

(30) Priority: 07.02.2005 KR 20050011478; 11.02.2005 KR 20050011631; 06.02.2006 KR 20060011083
(43) Date of publication of application: 24.10.2007
(60) Divisional application: 15188595.1
(73) Proprietor: Inktec Co., Ltd., Ansan-city, Kyeongki-do 425-839 (KR)
(72) Inventor: CHUNG, Kwang-Choon, Kangnam-gu, Seoul 135-270 (KR); CHO, Hyun-Nam, Gunpo-city, Kyeongki-do 435-040 (KR); GONG, Myoung-Seon, Kangnam-gu, Seoul 135-230 (KR); HAN, Yi-Sup, Goyang-city, Kyeongki-do 411-310 (KR); PARK, Jeong-Bin, Ansan-city Kyeongki-do 425-722 (KR); NAM, Dong Hun, Seoul 153-010 (KR); UHM, Seong-Yong, Suwon-city Kyeongki-do 440-710 (KR); SEO, Young-Kwan, Siheung-city, Kyeongki-go 429-702 (KR)
(74) Representative: Beckmann, Claus
(86) International application number: PCT/KR2006/000451
(87) International publication number: WO 2006/083153

(56) References cited:
- WO-A1-2006/093398
- WO-A2-2006/020584
- GB-A- 609 807
- US-A- 3 979 382
- US-A- 4 652 465
- US-A- 5 705 661
- US-A- 5 908 806
- US-A1- 2004 191 423

## Description

### Technical Field

The present invention relates to a novel organic silver complex prepared by reacting a silver compound with an ammonium carbamate compound or an ammonium carbonate compound and a preparation method thereof.

### Background Art

According to Ullmann's Encyclopedia of Ind. Chem., Vol. A24, 107(1993), silver is a precious metal resistant to oxidation, with superior electrical and thermal conductivity and catalytic and antibiotic activity. Thus, silver and silver compounds are widely used in alloys, plating, medicine, photography, electricity and electronics, fibers, detergents, household appliances, and so forth.

Silver compounds can be used as catalyst in synthesis of organic compounds and polymers. Especially, with the recent regulation of use of lead in electric and electronic circuits, use of silver in low-resistance metal wirings, printed circuit boards (PCB), flexible printed circuit boards (FPC), antennas for radio frequency identification (RFID) stags, plasma display panels (PDP), liquid crystal displays (TFT-LCD), organic light emitting diodes (OLED), flexible displays and organic thin-film transistors (OTFT) as metal patterns or electrodes is on the increase.

Mostly, silver is used in the form of a paste comprising silver powder, a binder and a solvent. Or, a silver compound such as silver nitrate is reacted with another compound in an aqueous solution or an organic solvent to obtain a variety of silver compounds or organic silver compounds containing nanoparticles. These organic silver compounds are used to form metal patterns by chemical vapor deposition (CVD), plasma vapor deposition, sputtering, electroplating, photolithography, electron beam technique, laser technique, etc.

The most common coordinator for organic silver complexes is carboxylic acid (Prog. Inorg. Chem., 10, p. 233(1968)). However, because silver-containing metal carboxylate complexes are generally sensitive to light, hardly soluble in organic solvents (J. Chem. Soc., (A)., p. 514 (1971), U.S. Patent No. 5,534,312 (July 9, 1996)) and have a high decomposition temperature, they are limited in application in spite of easiness in preparation. To solve this problem, several methods have been proposed in J. Inorg. Nucl. Chem., 40, p. 1599 (1978), Ang. Chem., Int. Ed. Engl., 31, p. 770 (1992), Eur. J. Solid State Inorg. Chem., 32, p. 25 (1995), J. Chem. Cryst., 26, p. 99 (1996), Chem. Vapor Deposition, 7, 111 (2001), Chem. Mater., 16, 2021 (2004), U.S. Patent No. 5,705,661 (January 6, 1998) and Korean Patent No. 2003-0085357 (November 5, 2003). Among them are the methods of using carboxylic acid compounds having long alkyl chains or including amine compounds or phosphine compounds. However, the silver derivatives known thus far are limited and have insufficient stability or solubility. Moreover, they have a high decomposition temperature to be applied for pattern formation and are decomposed slowly.

U.S. Patent No. 4,652,465 describes a process for producing a silver coated copper powder, characterized in that a metal silver is precipitated on the surface of a metal copper powder by means of a silver complex solution comprising a silver salt, an ammonium carbonate compound and ammonia water as essential components.

U.K. Patent No. 609,807 published in 1948 discloses a method of reacting ammonium carbonate or ammonium carbamate with a transition metal salt to obtain a transition metal salt coordinated by ammonia as carbon dioxide is generated. The patent mentions that silver complexes coordinated by ammonia can be prepared by the method. However, surprisingly, the present inventors found out that when ammonium carbonate or ammonium carbamate is added to a silver compound such as silver oxide, a stable silver complex is obtained without generation of carbon dioxide. They also confirmed that the silver complex is isolated as solid and can be easily prepared into thin film.

The silver complexes of the present invention are characterized in that, because they can be prepared under various reaction conditions, they have superior stability and solubility, can be easily prepared into thin film, thus enabling ease metal patterning, and are decomposed at low temperature, thus being easily prepared into thin film or powder.

### Disclosure of the Invention

It is an object of the present invention to provide a novel organic silver complex obtained by reacting a silver compound with an ammonium carbamate compound or an ammonium carbonate compound and a preparation method thereof.

It is another object of the present invention to provide a novel organic silver complex having superior stability and solubility and being easily prepared into thin film and a preparation method thereof.

It is still another object of the present invention to provide a novel organic silver complex which enables formation of high-purity metal film since it is decomposed at low temperature and a preparation method thereof.

### Brief Description of the Drawings

FIG. 1 is the ¹H NMR spectrum of the silver complex of Example 1.
FIG. 2 is the ¹³C NMR spectrum of the silver complex of Example 1.
FIG. 3 is the IR spectrum of the silver complex of Example 1.
FIG. 4 is the TGA thermogram of the silver complex of Example 1.
FIG. 5 is the DSC thermogram of the silver complex of Example 1.
FIG. 6 is the ¹H NMR spectrum of the silver complex of Example 23.
FIG. 7 is the ¹³C NMR spectrum of the silver complex of Example 23.
FIG. 8 is the IR spectrum of the silver complex of Example 23.
FIG. 9 is the TGA thermogram of the silver complex of Example 23.
FIG. 10 is the DSC thermogram of the silver complex of Example 23.
FIG. 11 is the ¹H NMR spectrum of the silver complex of Example 24.
FIG. 12 is the ¹³C NMR spectrum of the silver complex of Example 24.
FIG. 13 is the IR spectrum of the silver complex of Example 24.
FIG. 14 is the TGA thermogram of the silver complex of Example 24.
FIG. 15 is the DSC thermogram of the silver complex of Example 24.

### Best Mode for Carrying Out the Invention

In order to attain the objects, the present inventors invented novel organic silver complexes by reacting at least one silver compound represented by the formula 2 below with at least one ammonium carbamate compound or ammonium carbonate compound represented by the formula 3, 4 or 5 below:

AgₙX (2)

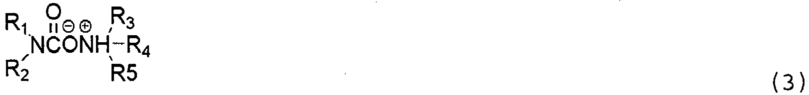

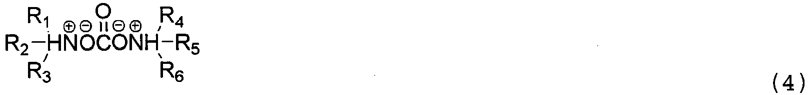

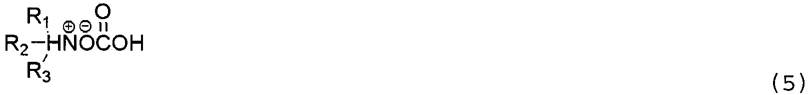

wherein, in the formula 2, n is an integer from 1 to 4, X is oxygen, sulfur, halogen, cyano, cyanate, carbonate, nitrate, nitrite, sulfate, phosphate, thiocyanate, chlorate, perchlorate, tetrafluoroborate, acetylacetonate or carboxylate (For example, the silver compound may be silver oxide, thiocyanate, silver sulfide, silver chloride, silver cyanide, silver cyanate, silver carbonate, silver nitrate, silver nitrite, silver sulfate, silver phosphate, silver perchlorate, silver tetrafluoroborate, silver acetylacetonate, silver acetate, silver lactate, silver oxalate or a derivative thereof. Silver oxide or silver carbonate is preferred with regard to reactivity or posttreatment, although not limited to them.), and
wherein the ammonium carbamate compound is selected from ethylammonium ethylcarbamate, isopropylammonium isopropylcarbamate, n-butylammonium n-butylcarbamate, isobutylammonium isobutylcarbamate, t-butylammonium t-butylcarbamate, 2-ethylhexylammonium 2-ethylhexylcarbamate, octadecylammonium octadecylcarbamate, 2-methoxyethylammonium 2-methoxyethylcarbamate, 2-cyanoethylammonium 2-cyanoethylcarbamate, dibutylammonium dibutylcarbamate, dioctadecylammonium dioctadecylcarbamate, methyldecylammonium methyldecylcarbamate, hexamethyleneiminium hexamethyleneiminecarbamate, morpholinium morpholinecarbamate, pyridinium ethylhexylcarbamate, triethylenediaminium isopropylbicarbamate, benzylammonium benzylcarbamate and triethoxysilylpropylammonium triethoxysilylpropylcarbamate.

For the ammonium carbamate compound of the present invention, one substituted by primary amine is preferred to those substituted by secondary or tertiary amine in terms of reactivity and stability.

The ammonium carbonate compound represented by the formula 4 or formula 5 is selected from ethylammonium ethylcarbonate, isopropylammonium isopropylcarbonate, isopropylammonium bicarbonate, *n*-butylammonium *n-*butylcarbonate, isobutylammonium isobutylcarbonate, *t-*butylammonium *t*-butylcarbonate, *t*-butylammonium bicarbonate, 2-ethylhexylammonium 2-ethylhexylcarbonate, 2-ethylhexylammonium bicarbonate, 2-methoxyethylammonium 2-methoxyethylcarbonate, 2-methoxyethylammonium bicarbonate, 2-cyanoethylammonium 2-cyanoethylcarbonate, 2-cyanoethylammonium bicarbonate, octadecylammonium octadecylcarbonate, dibutylammonium dibutylcarbonate, dioctadecylammonium dioctadecylcarbonate, dioctadecylammonium bicarbonate, methyldecylammonium methyldecylcarbonate, hexamethyleneiminium hexamethyleneiminecarbonate, morpholinium morpholinecarbonate, benzylammonium benzylcarbonate, triethoxysilylpropylammonium triethoxysilylpropylcarbonate, pyridinium bicarbonate, triethylenediaminium isopropylcarbonate and triethylenediaminium bicarbonate.

The silver complex can also be obtained by reacting:
- silver oxide with n-propylammonium n-propylcarbamate,
- silver oxide with 2-hydroxyethylammonium 2-hydroxyethylcarbamate,
- silver oxide with aminoethylammonium aminoethylcarbamate,
- silver oxide with 2-ethylhexylammonium 2-ethylhexylcarbamate and aminoethylammonium aminoethylcarbamate,
- silver oxide with octylammonium bicarbonate,
- silver oxide with isobutylammonium bicarbonate,
- silver oxide with n-butylammonium bicarbonate,
- silver oxide with morpholinium bicarbonate,
- silver oxide with aminoethylammonium aminoethylcarbonate,
- silver oxide with 2-ethylhexylammonium 2-ethylhexylcarbonate and aminoethylammonium aminoethylcarbonate,
- silver oxide with ammonium carbamate,
- silver carbonate with ammonium carbamate,
- silver oxide with ammonium carbonate,
- silver carbonate with ammonium carbonate,
- silver oxide with ammonium bicarbonate, or
- silver carbonate with ammonium bicarbonate.

The preparation method of the ammonium carbamate compound and the ammonium carbonate compound is not particularly limited. For example, J. Am. Chem. Soc., 70, p. 3865 (1948), J. Am. Chem. Soc., 73, p. 1829 (1951), J. Prakt. Chem., 9, p. 217 (1959), J. Am. Chem. Soc. , 123, p. 10393 (2001), Langmuir, 18, 7124 (2002) and U.S. Patent No. 4,542,214 (September 17, 1985) disclose that the compounds can be prepared from primary amine, secondary amine, tertiary amine or a mixture thereof and carbon dioxide. According to the disclosure, an ammonium carbonate compound is obtained if 0.5 mole of carbon dioxide is used per 1 mole of amine and an ammonium bicarbonate compound is obtained if more than 1 mole of carbon dioxide is used per 1 mole of amine. The preparation may be performed under normal pressure or applied pressure with or without a solvent. When a solvent is used, an alcohol such as methanol, ethanol, isopropanol and butanol, a glycol such as ethylene glycol and glycerine, an acetate such as ethyl acetate, butyl acetate and carbitol acetate, an ether such as diethyl ether, tetrahydrofuran and dioxane, a ketone such as methyl ethyl ketone and acetone, a hydrocarbon solvent such as hexane and heptane, an aromatic solvent such as benzene and toluene, a halogen-substituted solvent such as chloroform, methylene chloride and carbon tetrachloride, etc. may be used. Carbon dioxide may be bubbled in the gas phase or solid dry ice may be used. The reaction may be performed in the supercritical state. Any other known methods can be applied for the preparation of the ammonium carbamate derivative and the ammonium carbonate derivative, as long as the structure of the target compound is the same. That is, preparation solvent, reaction temperature, concentration, catalyst, etc. are not particularly limited. And, the preparation yield is irrelevant of the preparation method.

Such prepared ammonium carbamate compound or ammonium carbonate compound is reacted with the silver compound to obtain the organic silver complex. For example, at least one silver compound represented by the formula 2 may be reacted with at least one ammonium carbamate derivative or ammonium carbonate derivative represented by the formulas 3 to 5 under nitrogen atmosphere at normal pressure or applied pressure with or without a solvent. When a solvent is used, an alcohol such as methanol, ethanol, isopropanol and butanol, a glycol such as ethylene glycol and glycerine, an acetate such as ethyl acetate, butyl acetate and carbitol acetate, an ether such as diethyl ether, tetrahydrofuran and dioxane, a ketone such as methyl ethyl ketone and acetone, a hydrocarbon solvent such as hexane and heptane, an aromatic solvent such as benzene and toluene, halogen-substituted solvent such as chloroform, methylene chloride and carbon tetrachloride, etc. may be used. However, the solvent used in the preparation of the organic silver complex of the present invention needs not be particularly limited. That is, any other solvent may be used as long as the structure of the target compound is the same.

The silver complex of the present invention has the structure represented by the following formula 1:

Ag[A]ₘ (1)

where A is the compound represented by the formula 3, 4 or 5 and 0.7 ≤ m ≤ 2.5.

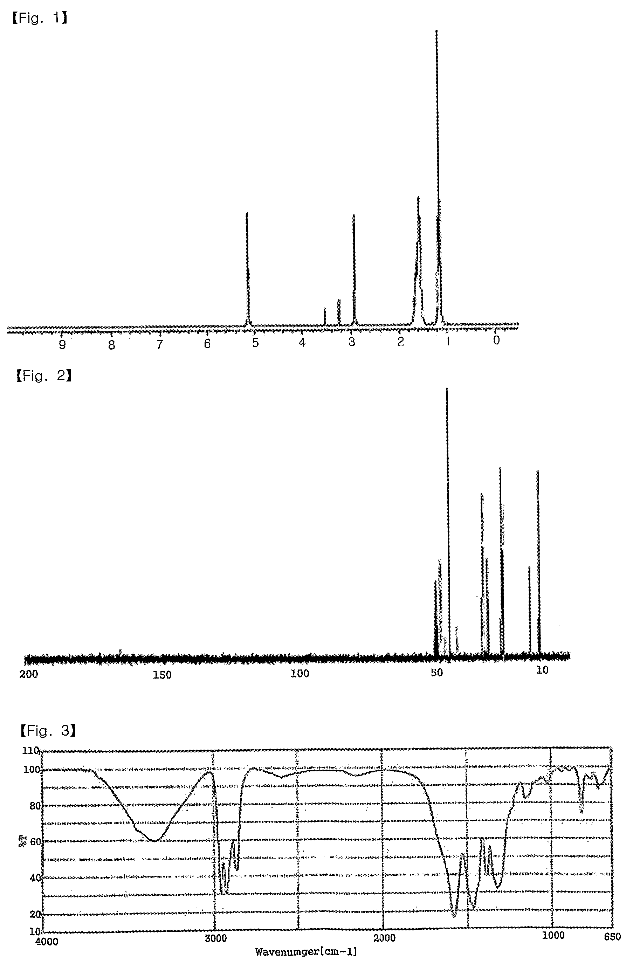

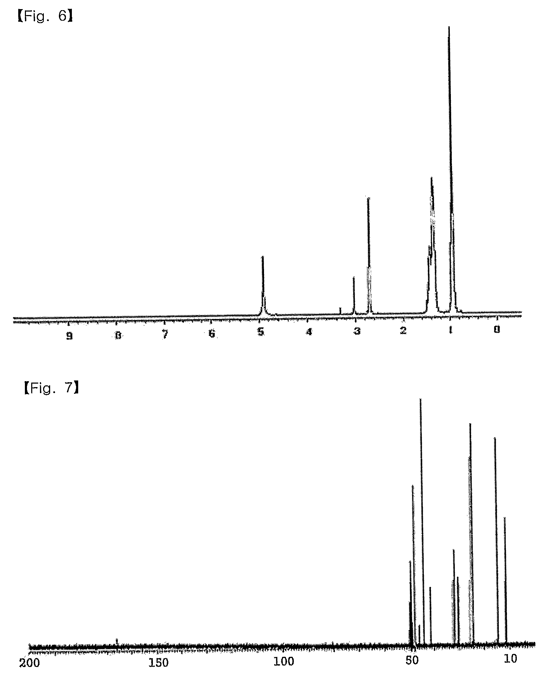

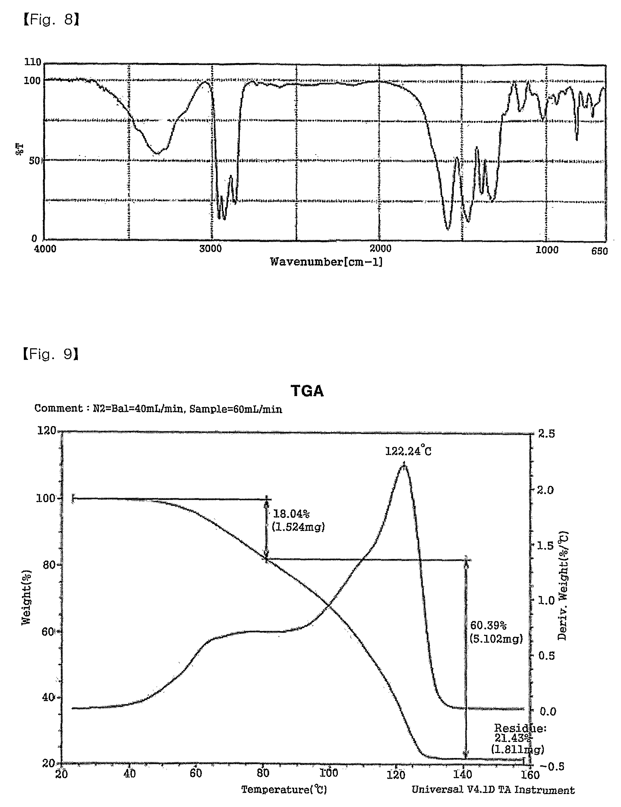

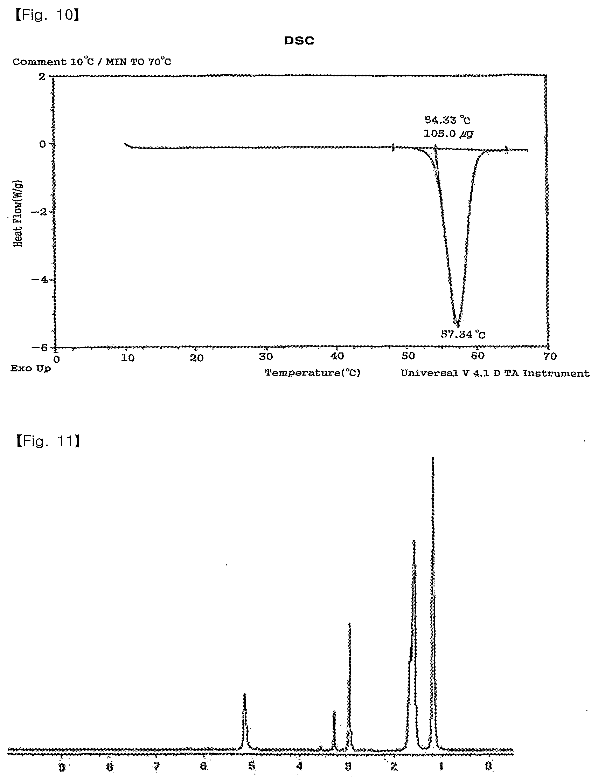

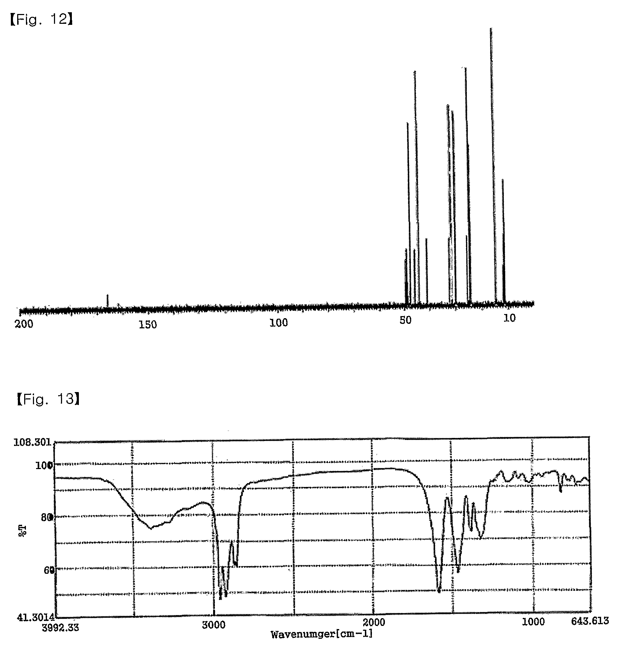

The silver complexes of the present invention are isolated as white solid. When decomposed by heating, the resultant compounds contain conductive, non-valent silver, not in the oxidized state. The IR spectrums (FIG. 3, FIG. 8 and FIG. 13) of the silver complexes show C=O absorption bands and confirms that carbon dioxide is not produced. The ¹H NMR spectrums (FIG. 1, FIG. 6 and FIG. 11) and the ¹³C NMR spectrums (FIG. 2, FIG. 7 and FIG. 12) also confirm the functional groups of the ammonium carbamate compound or the ammonium carbonate compound.

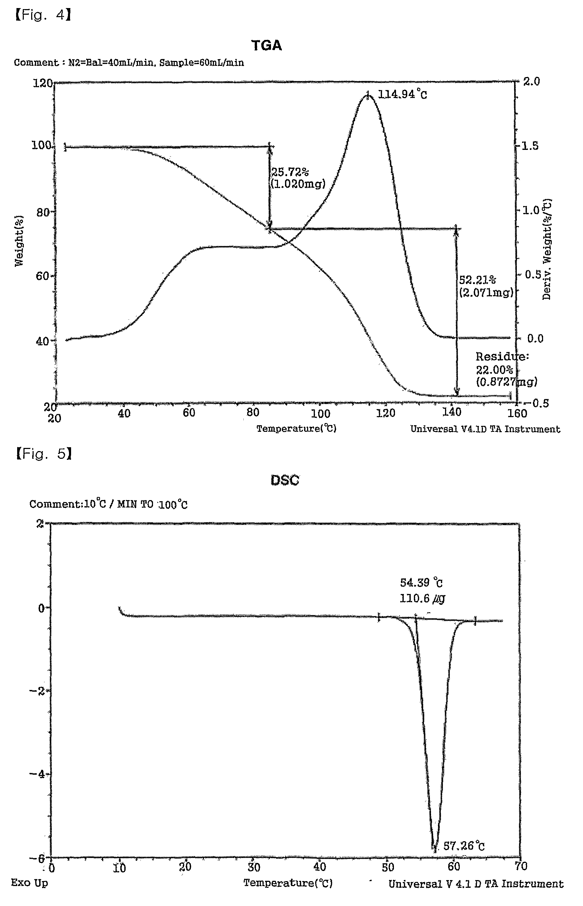

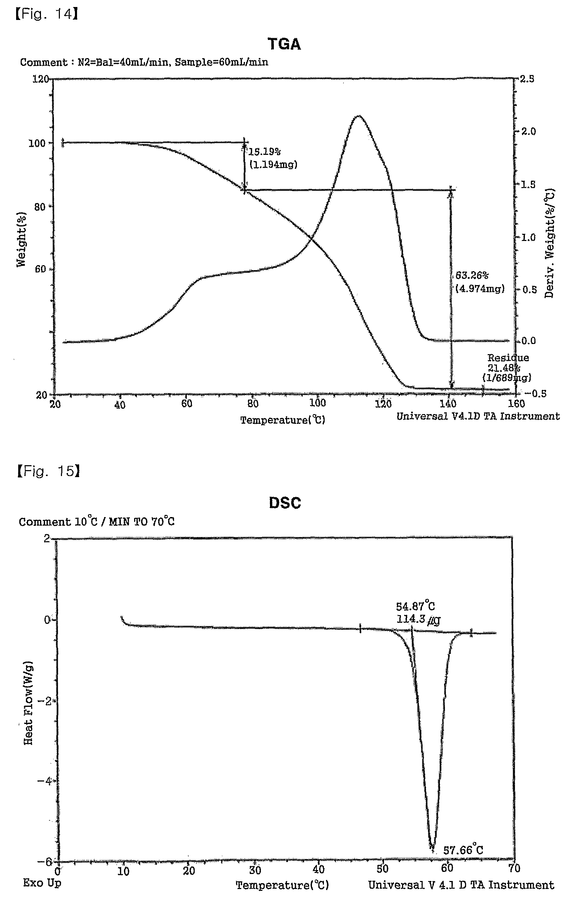

The silver complexes of the present invention show specific melting points and decomposition patterns as seen in the TGA thermograms and the DSC thermograms (FIG. 4, FIG. 5, FIG. 9, FIG. 10, FIG. 14 and FIG. 15). When the melt silver complexes are cooled, stable solid silver complexes are obtained.

The organic silver complex of the present invention is highly soluble in a variety of solvents including ones used to prepare the organic silver complex, for example, an alcohol such as methanol, an ester such as ethyl acetate, an ether such as tetrahydrofuran, etc. Thus, the silver complex can be readily used in coating or printing and can be stably stored in the form of solution for over 3 months.

The organic silver complex solution may be prepared into thin film by coating on a substrate of glass, silicon wafer, polymer film like polyester and polyimide, paper, etc. or printed directly. The thin film formation may be performed by spin coating, roll coating, spray coating, dip coating, flow coating, etc. And, the printing may be performed by ink-jet printing, offset printing, screen printing, gravure printing, flexo printing, etc.

The prepared thin film may be oxidized, reduced or heat-treated or the organic silver complex may be treated with chemical vapor deposition (CVD), plasma vapor deposition, sputtering, electroplating, lithography, IR, electron beam or laser to obtain a metal or metal oxide pattern. The heat treatment may be performed under inert gas atmosphere, as usually, but also may be performed in air or using a mixture gas of hydrogen and air or other inert gas.

Hereinafter, the present invention is described in further detail through examples. However, the following examples are only for the understanding of the present invention and the present invention is not limited to or by them.

### Examples

### Example 1: Reaction of silver oxide with 2-ethylhexylammonium 2-ethylhexylcarbamate

In a 50 mL Schlenk flask equipped with a stirrer, 3.25 g (10.75 mmol) of 2-ethylhexylammonium 2-ethylhexylcarbamate (viscous liquid) was dissolved in 10 mL of methanol. 1.0 g (4.31 mmol) of silver oxide was added and reaction was performed at room temperature. The reaction solution was initially a black slurry but it turned transparent as complex was produced. After 2 hours of reaction, a colorless, transparent solution was obtained. The resultant solution was filtered with a 0.45 micron membrane filter to remove unreacted silver oxide. Then, the solvent was removed at vacuum to obtain white solid. The solid was recrystallized in ethyl acetate, dried and weighed to obtain 4.22 g of a silver complex (yield = 99.4 %). The silver complex had a melting point of 57-58 °C (DSC = 57.26 °C) and a silver content of 22.0 wt% (TGA analysis).
¹H NMR (CD₃OD, ppm), 1.11-1.19(m, -CH₃) 1.51-1.69(m, -CH₂,-CH), 2.91-2.92, 3.23-3.25(d, -NCH₂), 5.13(s, -NH₂), ¹³C NMR (CD₃OD, ppm), 166.09, 47.60, 44.24, 31.76, 30.12, 24.77, 24.30, 14.64, 11.15

### Example 2: Reaction of silver oxide with n-propylammonium n-propylcarbamate

In a 50 mL Schlenk flask equipped with a stirrer, 1.74 g (10.75 mmol) of n-propylammonium n-propylcarbamate (viscous liquid, melting point: 74-76 °C) was dissolved in 10 mL of methanol. 1.0 g (4.31 mmol) of silver oxide was added and reaction was performed at room temperature for 2 hours while stirring. A colorless, transparent complex solution was obtained as in Example 1. The resultant solution was filtered with a 0.45 micron membrane filter to remove and the solvent was removed at vacuum to obtain white solid. The solid was dried and weighed to obtain 2.42 g of a silver complex (yield = 88.3 %). Most of the silver complex was decomposed below 130 °C to leave metallic silver. The silver content was 38.4 wt% (TGA analysis).
¹H NMR (CD₃OD, ppm), 0.98-1.02(t, -CH₃) 1.59-1.65 (m, -CH₂), 2.76-2.80(t, -NCH₂), ¹³C NMR (CD₃OD, ppm), 47.03, 27.84, 11.53

### Example 3: Reaction of silver oxide with isopropylammonium isopropylcarbamate

In a 50 mL Schlenk flask equipped with a stirrer, 1.60 g (10.75 mmol) of isopropylammonium isopropylcarbamate (white solid, melting point: 78-80 °C) was dissolved in 10 mL of methanol. 1.0 g (4.31 mmol) of silver oxide was added and reaction was performed at room temperature. The reaction solution was initially a black slurry but it turned transparent as complex was produced. After 2 hours of reaction, a colorless, transparent solution was obtained. The resultant solution was filtered with a 0.45 micron membrane filter and the solvent was removed at vacuum to obtain white solid. The solid was dried and weighed to obtain 2.48 g of a silver complex (yield = 95.5 %). Most of the silver complex was decomposed below 130 °C to leave metallic silver. The silver content was 37.2 wt% (TGA analysis).
¹H NMR (CD₃OD, ppm), 1.13-1.22(d, -CH₃), 3.22-3.31(m, CH), ¹³C NMR (CD₃OD, ppm), 45.78, 26.06

### Example 4: Reaction of silver oxide with n-butylammonium n-butylcarbamate

In a 50 mL Schlenk flask equipped with a stirrer, 2.04 g (10.75 mmol) of n-butylammonium n-butylcarbamate (white solid, melting point: 82-84 °C) was dissolved in 10 mL of methanol. 1.0 g (4.31 mmol) of silver oxide was added and reaction was performed at room temperature. The reaction solution was initially a black slurry but it turned transparent as complex was produced. After 2 hours of reaction, a colorless, transparent solution was obtained. The resultant solution was filtered with a 0.45 micron membrane filter and the solvent was removed at vacuum to obtain white solid. The solid was dried and weighed to obtain 2.79 g of a silver complex (yield = 92.0 %). Most of the silver complex was decomposed below 130 °C to leave metallic silver. The silver content was 33.2 wt% (TGA analysis).
¹H NMR (CD₃OD, ppm), 0.92-0.97(t, -CH₃), 1.37-1.46(m, -CH₂), 1.52-1.59(m, -CH₂), 2.75-2.79 (t, -NCH₂), ¹³C NMR (CD₃OD, ppm), 161.46, 44.76, 36.94, 21.05, 14.38

### Example 5: Reaction of silver oxide with isobutylammonium isobutylcarbamate

In a 50 mL Schlenk flask equipped with a stirrer, 2.04 g (10.75 mmol) of isobutylammonium isobutylcarbamate (white solid, melting point: 80-82 °C) was dissolved in 10 mL of methanol. 1.0 g (4.31 mmol) of silver oxide was added and reaction was performed at room temperature. The reaction solution was initially a black slurry but it turned transparent as complex was produced. After 2 hours of reaction, a colorless, transparent solution was obtained. The resultant solution was filtered with a 0.45 micron membrane filter and the solvent was removed at vacuum to obtain white solid. The solid was dried and weighed to obtain 2.87 g of a silver complex (yield = 94.4 %). Most of the silver complex was decomposed below 130 °C to leave metallic silver. The silver content was 32.4 wt% (TGA analysis).
¹H NMR (CD₃OD, ppm), 0.96-0.98(d, -CH₃), 1.67-1.74(m, -CH), 2.59-2.88(dd, -CH₂), ¹³C NMR (CD₃OD, ppm), 161.48, 52.69, 33.16, 30.45, 20.42

### Example 6: Reaction of silver oxide with t-butylammonium t-butylcarbamate

In a 50 mL Schlenk flask equipped with a stirrer, 2.04 g (10.75 mmol) of t-butylammonium t-butylcarbamate (white solid) was dissolved in 10 mL of methanol. 1.0 g (4.31 mmol) of silver oxide was added and reaction was performed at room temperature. The reaction solution was initially a black slurry but it turned transparent as complex was produced. After 2 hours of reaction, a colorless, transparent solution was obtained. The resultant solution was filtered with a 0.45 micron membrane filter and the solvent was removed at vacuum to obtain white solid. The solid was dried and weighed to obtain 2.94 g of a silver complex (yield = 97.0 %). Most of the silver complex was decomposed below 130 °C to leave metallic silver. The silver content was 31.4 wt% (TGA analysis).
¹H NMR (CD₃OD, ppm), 1.27 (s, -CH₃), ¹³C NMR (CD₃OD, ppm), 161.52, 50.94, 32.28

### Example 7: Reaction of silver carbonate with 2-ethylhexylammonium 2-ethylhexylcarbamate

In a 50 mL Schlenk flask equipped with a stirrer, 3.27 g (10.80 mmol) of 2-ethylhexylammonium 2-ethylhexylcarbamate (viscous liquid) was dissolved in 10 mL of methanol and 1.0 g (3.60 mmol) of silver carbonate was added. The reaction solution was initially a yellow slurry but it turned transparent as reaction proceeded. After 5 hours of reaction, a yellow, transparent solution was obtained, which confirmed production of a complex. The resultant solution was filtered with a 0.45 micron membrane filter and the solvent was removed at vacuum to obtain white solid. The solid was dried and weighed to obtain 4.18 g of a silver complex (yield = 97.89 %). Most of the silver complex was decomposed below 130 °C to leave metallic silver. The silver content was 18.66 wt% (TGA analysis).
¹H NMR (CD₃OD, ppm), 1.11-1.19(m, -CH₃) 1.51-1.69(m, -CH₂,-CH-), 2.91-2.92, 3.23-3.25 (d, -NCH₂), 5.13(t, -NHₓ), ¹³C NMR (CD₃OD, ppm), 166.09, 47.60, 44.24, 31.76, 30.12, 24.77, 24.30, 14.64, 11.15

### Example 8: Reaction of silver oxide with 2-methoxyethylammonium 2-methoxyethylcarbamate

In a 50 mL Schlenk flask equipped with a stirrer, 2.17 g (11.18 mmol) of 2-methoxyethylammonium 2-methoxyethylcarbamate (white solid, melting point: 41-42 °C) was dissolved in 10 mL of methanol. 1.0 g (4.31 mmol) of silver oxide was added and reaction was performed at room temperature. The reaction solution was initially a black slurry but it turned transparent as complex was produced. After 2 hours of reaction, a yellow, transparent solution was obtained. The resultant solution was filtered with a 0.45 micron membrane filter and the solvent was removed at vacuum to obtain brown, viscous liquid. The liquid was dried and weighed to obtain 2.58 g of a silver complex (yield = 81.4 %). Most of the silver complex was decomposed below 130 °C to leave metallic silver. The silver content was 35.9 wt% (TGA analysis).
¹H NMR (CD₃OD, ppm), 2.93-2.96(t, -NCH₂), 3.39(s, -OCH₃), 3.48-3.50(t, OCH₂), ¹³C NMR (CD₃OD, ppm), 161.48, 74.11, 59.35, 44.34

### Example 9: Reaction of silver oxide with 2-hydroxyethylammonium 2-hydroxyethylcarbamate

In a 50 mL Schlenk flask equipped with a stirrer, 1.78 g (12.90 mmol) of 2-hydroxyethylammonium 2-hydroxyethylcarbamate was dissolved in 10 mL of methanol. 1.0 g (4.31 mmol) of silver oxide was added and reaction was performed at room temperature. The reaction solution was initially a black slurry but it turned transparent as complex was produced. After 2 hours of reaction, a yellow, transparent solution was obtained. The resultant solution was filtered with a 0.45 micron membrane filter and the solvent was removed at vacuum to obtain brown, viscous liquid. The liquid was dried and weighed to obtain 2.50 g of a silver complex (yield = 90.1 %). Most of the silver complex was decomposed below 130 °C to leave metallic silver. The silver content was 37.1 wt% (TGA analysis). ¹H NMR (CD₃OD, ppm), 2.82-2.85(t, -NCH₂), 3.61-3.64(t, OCH₂), ¹³C NMR (CD₃OD, ppm), 166.16, 63.70, 46.12

### Example 10: Reaction of silver oxide with 2-cyanoethylammonium 2-cyanoethylcarbamate

In a 50 mL Schlenk flask equipped with a stirrer, 2.40 g (12.90 mmol) of 2-cyanoethylammonium 2-cyanoethylcarbamate (white solid, melting point: 70-72 °C) was dissolved in 10 mL of methanol. 1.0 g (4.31 mmol) of silver oxide was added and reaction was performed at room temperature. The reaction solution was initially a black slurry but it turned transparent as complex was produced. After 2 hours of reaction, a colorless, transparent solution was obtained. The resultant solution was filtered with a 0.45 micron membrane filter and the solvent was removed at vacuum to obtain white solid. The solid was dried and weighed to obtain 3.06 g of a silver complex (yield = 90.0 %). About 60 % of the silver complex was decomposed below 150 °C to leave metallic silver and unreacted organic materials. Most of the silver complex was decomposed below 250 °C to leave metallic silver. The silver content was 28.7 wt% (TGA analysis).

### Example 11: Reaction of silver oxide with morpholinium morpholinecarbamate

In a 50 mL Schlenk flask equipped with a stirrer, 2.81 g (12.90 mmol) of morpholinium morpholinecarbamate was dissolved in 10 mL of methanol. 1.0 g (4.31 mmol) of silver oxide was added and reaction was performed at room temperature. The reaction solution was initially a black slurry but it turned transparent as complex was produced. After 2 hours of reaction, a yellow, transparent solution was obtained. The resultant solution was filtered with a 0.45 micron membrane filter and the solvent was removed at vacuum to obtain gray solid. The solid was dried and weighed to obtain 3.29 g of a silver complex (yield = 86.4 %). Most of the silver complex was decomposed below 130 °C to leave metallic silver. The silver content was 28.3 wt% (TGA analysis).

### Example 12: Reaction of silver oxide with hexamethyleneiminium hexamethyleneiminecarbamate

In a 50 mL Schlenk flask equipped with a stirrer, 3.13 g (12.90 mmol) of hexamethyleneiminium hexamethyleneiminecarbamate was dissolved in 10 mL of methanol. 1.0 g (4.31 mmol) of silver oxide was added and reaction was performed at room temperature. The reaction solution was initially a black slurry but it turned transparent as complex was produced. After 2 hours of reaction, a yellow, transparent solution was obtained. The resultant solution was filtered with a 0.45 micron membrane filter and the solvent was removed at vacuum to obtain brown liquid. The liquid was dried and weighed to obtain 3.29 g of a silver complex (yield = 86.8 %). Most of the silver complex was decomposed below 130 °C to leave metallic silver. The silver content was 25.9 wt% (TGA analysis).

### Example 13: Reaction of silver oxide with ammonium carbamate

In a 250 mL Schlenk flask equipped with a stirrer, 6.71 g (86 mmol) of ammonium carbamate and 15 g of isopropylamine (0.25 mol) were dissolved in 50 mL of methanol. 10.0 g (43.1 mmol) of silver oxide was added and reaction was performed at room temperature. The reaction solution was initially a black slurry but it turned transparent as complex was produced. After 3 hours of reaction, a colorless, transparent solution was obtained. The resultant solution was filtered with a 0.45 micron membrane filter and the solvent was removed at vacuum to obtain white solid. The solid was dried and weighed to obtain 26.90 g of a silver complex (yield = 84.9 %). Most of the silver complex was decomposed below 130 °C to leave metallic silver. The silver content was 42.0 wt% (TGA analysis).

### Example 14: Reaction of silver carbonate with ammonium carbamate

In a 250 mL Schlenk flask equipped with a stirrer, 3.36 g (43 mmol) of ammonium carbamate and 15 g of isopropylamine (0.25 mol) were dissolved in 50 mL of methanol. 11.88 g (43.1 mmol) of silver carbonate was added and reaction was performed at room temperature. The reaction solution was initially a yellow slurry but it turned transparent as complex was produced. After 6 hours of reaction, a colorless, transparent solution was obtained. The resultant solution was filtered with a 0.45 micron membrane filter and the solvent was removed at vacuum to obtain white solid. The solid was dried and weighed to obtain 25.60 g of a silver complex (yield = 84.5 %). Most of the silver complex was decomposed below 130 °C to leave metallic silver. The silver content was 45.8 wt% (TGA analysis).

### Example 15: Reaction of silver oxide with 2-ethylhexylammonium 2-ethylhexylcarbamate

In a 50 mL Schlenk flask equipped with a stirrer, 3.25 g (10.75 mmol) of 2-ethylhexylammonium 2-ethylhexylcarbamate (viscous liquid) was dissolved in 10 mL of tetrahydrofuran (THF). 1.0 g (4.31 mmol) of silver oxide was added and reaction was performed at room temperature. The reaction solution was initially a black slurry but it turned transparent as complex was produced. After 2 hours of reaction, a yellow, transparent solution was obtained. The resultant solution was filtered with a 0.45 micron membrane filter and the solvent was removed at vacuum to obtain white solid. The solid was dried and weighed to obtain 3.58 g of a silver complex (yield = 88.23 %). Most of the silver complex was decomposed below 130 °C to leave metallic silver. The silver content was 25.97 wt% (TGA analysis).

### Example 16: Reaction of silver oxide with 2-ethylhexylammonium 2-ethylhexylcarbamate

In a 50 mL Schlenk flask equipped with a stirrer, 3.25 g (10.75 mmol) of 2-ethylhexylammonium 2-ethylhexylcarbamate (viscous liquid) was dissolved in 10 mL of ethyl acetate. 1.0 g (4.31 mmol) of silver oxide was added and reaction was performed at room temperature. The reaction solution was initially a black slurry but it turned transparent as complex was produced. After 2 hours of reaction, a colorless, transparent solution was obtained. The resultant solution was filtered with a 0.45 micron membrane filter and the solvent was removed at vacuum to obtain white solid. The solid was dried and weighed to obtain 3.53 g of a silver complex (yield = 83.17 %). Most of the silver complex was decomposed below 130 °C to leave metallic silver. The silver content was 26.34 wt% (TGA analysis).

### Example 17: Reaction of silver oxide with 2-ethylhexylammonium 2-ethylhexylcarbamate

To a 50 mL Schlenk flask equipped with a stirrer was added 3.90 g (12.90 mmol) of 2-ethylhexylammonium 2-ethylhexylcarbamate (viscous liquid). 1.0 g (4.31 mmol) of silver oxide was added and reaction was performed at room temperature. The reaction solution was initially a black slurry but it turned transparent as complex was produced. After 2 hours of reaction, a yellow, transparent solution was obtained. The resultant solution was filtered with a 0.45 micron membrane filter and kept at vacuum to obtain white solid. The solid was dried and weighed to obtain 3.58 g of a silver complex (yield = 88.23 %). Most of the silver complex was decomposed below 130 °C to leave metallic silver. The silver content was 25.97 wt% (TGA analysis).

### Example 18: Reaction of silver oxide with aminoethylammonium aminoethylcarbamate

In a 50 mL Schlenk flask equipped with a stirrer, 1.763 g (10.75 mmol) of aminoethylammonium aminoethylcarbamate (white solid) was dissolved in 10 mL of methanol. 1.0 g (4.31 mmol) of silver oxide was added and reaction was performed at room temperature. The reaction solution was initially a black slurry but it turned transparent as complex was produced. After 2 hours of reaction, a colorless, transparent solution was obtained. The resultant solution was filtered with a 0.45 micron membrane filter and the solvent was removed at vacuum to obtain black, viscous liquid. The liquid was dried and weighed to obtain 2.21 g of a silver complex (yield = 79.99 %). Most of the silver complex was decomposed below 130 °C to leave metallic silver. The silver content was 42.12 wt% (TGA analysis).

### Example 19: Reaction of silver oxide with 2-ethylhexylammonium 2-ethylhexylcarbamate and aminoethylammonium aminoethylcarbamate

In a 50 mL Schlenk flask equipped with a stirrer, 3.07 g (10.80 mmol) of a 6:1 (molar ratio) mixture of 2-ethylhexylammonium 2-ethylhexylcarbamate and aminoethylammonium aminoethylcarbamate was dissolved in 10 mL of methanol. 1.0 g (4.31 mmol) of silver oxide was added and reaction was performed at room temperature. The reaction solution was initially a black slurry but it turned transparent as complex was produced. After 2 hours of reaction, a colorless, transparent solution was obtained. The resultant solution was filtered with a 0.45 micron membrane filter and the solvent was removed at vacuum to obtain orange, viscous liquid. The liquid was dried and weighed to obtain 3.85 g of a silver complex (yield = 94.59 %). Most of the silver complex was decomposed below 130 °C to leave metallic silver. The silver content was 24.20 wt% (TGA analysis).

### Example 20: Reaction of silver sulfate with 2-ethylhexylammonium 2-ethylhexylcarbamate

In a 50 mL Schlenk flask equipped with a stirrer, 2.42 g (8.00 mmol) of 2-ethylhexylammonium 2-ethylhexylcarbamate (viscous liquid) was dissolved in 10 mL of methanol. 1.0 g (3.2 mmol) of silver sulfate was added and reaction was performed at room temperature. The reaction solution was initially a white slurry but it turned transparent as complex was produced. After 2 hours, a completely transparent solution was obtained. The resultant solution was filtered with a 0.45 micron membrane filter and the solvent was removed at vacuum to obtain white solid. The solid was recrystallized in ethyl acetate, dried and weighed to obtain 3.15 g of a silver complex (yield = 92.3 %). Most of the silver complex was decomposed below 130 °C to leave metallic silver. The silver content was 21.35 wt% (TGA analysis).

### Example 21: Reaction of silver nitrate with 2-ethylhexylammonium 2-ethylhexylcarbamate

In a 50 mL Schlenk flask equipped with a stirrer, 2.23 g (7.37 mmol) of 2-ethylhexylammonium 2-ethylhexylcarbamate (viscous liquid) was dissolved in 10 mL of methanol. 1.0 g (5.9 mmol) of silver nitrate was added and reaction was performed at room temperature. The reaction solution was initially a white slurry but it turned transparent as complex was produced. After 2 hours, a completely transparent solution was obtained. The resultant solution was filtered with a 0.45 micron membrane filter and the solvent was removed at vacuum to obtain white solid. The solid was recrystallized in ethyl acetate, dried and weighed to obtain 2.76 g of a silver complex (yield = 85.6 %). Most of the silver complex was decomposed below 130 °C to leave metallic silver. The silver content was 22.73 wt% (TGA analysis).

### Example 22: Reaction of silver cyanide with 2-ethylhexylammonium 2-ethylhexylcarbamate

In a 50 mL Schlenk flask equipped with a stirrer, 2.83 g (9.37 mmol) of 2-ethylhexylammonium 2-ethylhexylcarbamate (viscous liquid) was dissolved in 10 mL of dimethylsulfoxide (DMSO). 1 g (7.5 mmol) of silver cyanide was added and reaction was performed at room temperature. The reaction solution was initially a white slurry but it turned transparent as complex was produced. After 2 hours, a completely transparent solution was obtained. The resultant solution was filtered with a 0.45 micron membrane filter and the solvent was removed at vacuum to obtain white solid. The solid was recrystallized in ethyl acetate, dried and weighed to obtain 3.15 g of a silver complex (yield = 82.42 %). Most of the silver complex was decomposed below 130 °C to leave metallic silver. The silver content was 25.43 wt% (TGA analysis).

### Example 23: Reaction of silver oxide with 2-ethylhexylammonium 2-ethylhexylcarbonate

In a 50 mL Schlenk flask equipped with a stirrer, 3.72 g (11.61 mmol) of 2-ethylhexylammonium 2-ethylhexylcarbamate (viscous liquid) was dissolved in 10 mL of methanol. 1.0 g (4.31 mmol) of silver oxide was added and reaction was performed at room temperature. The reaction solution was initially a black slurry but it turned transparent as complex was produced. After 2 hours, a colorless, transparent solution was obtained. The resultant solution was filtered with a 0.45 micron membrane filter to remove unreacted silver oxide particles and the solvent was removed at vacuum to obtain white solid. The solid was dried and weighed to obtain 4.02 g of a silver complex (yield = 85.2 %). The silver complex had a melting point of 55-57 °C (DSC = 57.34 °C) and a silver content of 21.43 wt% (TGA analysis).
¹H NMR (CD₃OD, ppm), 0.87-0.99(m, -CH₃) 1.31-1.47(m, -CH₂,-CH-), 2.69-2.70, 3.01-3.02(d, -NCH₂), 4.90(s, -NH₂), ¹³C NMR (CD₃OD, ppm), 165.00, 47.70, 44.25, 31.73, 30.90, 24.73, 24.29, 14.68, 11.16

### Example 24: Reaction of silver oxide with 2-ethylhexylammonium bicarbonate

In a 50 mL Schlenk flask equipped with a stirrer, 4.86 g (25.37 mmol) of 2-ethylhexylammonium bicarbonate (viscous liquid) was dissolved in 10 mL of methanol. 1.0 g (4.31 mmol) of silver oxide was added and reaction was performed at room temperature. The reaction solution was initially a black slurry but it turned transparent as complex was produced. After 2 hours, a colorless, transparent solution was obtained. The resultant solution was filtered with a 0.45 micron membrane filter and the solvent was removed at vacuum to obtain white solid. The solid was dried and weighed to obtain 4.33 g of a silver complex (yield = 73.9 %). The silver complex had a melting point of 56-57 °C (DSC = 57.66 °C) and a silver content of 21.48 wt% (TGA analysis).
¹H NMR (CD₃OD, ppm), 0.93-1.08(m, -CH₃) 1.31-1.64(m, -CH₂,-CH-), 2.93-2.94, 3.25-3.26(d, -NCH₂), 5.13(s, -NH₂), ¹³C NMR (CD₃OD, ppm), 165.56, 47.73, 44.23, 31.713, 30.08, 24.72, 24.28, 14.69, 11.17

### Example 25: Reaction of silver oxide with isopropylammonium isopropylcarbonate

In a 50 mL Schlenk flask equipped with a stirrer, 2.01 g (11.18 mmol) of isopropylammonium isopropylcarbonate was dissolved in 10 mL of methanol and 1.0 g (4.31 mmol) of silver oxide was added. The reaction solution was initially a black slurry but it turned transparent as complex was produced. After 2 hours, a colorless, transparent solution was obtained. The resultant solution was filtered with a 0.45 micron membrane filter and the solvent was removed at vacuum to obtain white solid. The solid was dried and weighed to obtain 2.41 g of a silver complex (yield = 80.2 %). Most of the silver complex was decomposed below 130 °C and the silver content was 38.6 wt% (TGA analysis).

### Example 26: Reaction of silver carbonate with isopropylammonium isopropylcarbonate

In a 50 mL Schlenk flask equipped with a stirrer, 2.07 g (11.52 mmol) of isopropylammonium isopropylcarbonate was dissolved in 10 mL of methanol and 1.0 g (3.60 mmol) of silver carbonate was added. The reaction solution was initially a yellow slurry but it turned transparent as complex was produced. After 6 hours, a colorless, transparent solution was obtained. The resultant solution was filtered with a 0.45 micron membrane filter and the solvent was removed at vacuum to obtain white solid. The solid was dried and weighed to obtain 2.42 g of a silver complex (yield = 78.8 %). Most of the silver complex was decomposed below 130 °C and the silver content was 32.23 wt% (TGA analysis).

### Example 27: Reaction of silver carbonate with 2-ethylhexylammonium 2-ethylhexylcarbonate

In a 50 mL Schlenk flask equipped with a stirrer, 3.46 g (14.4 mmol) of 2-ethylhexylammonium 2-ethylhexylcarbonate (viscous liquid) was dissolved in 10 mL of methanol and 1.0 g (3.60 mmol) of silver carbonate was added. The reaction solution was initially a yellow slurry but it turned transparent as complex was produced. After 6 hours, a yellow, transparent solution was obtained. The resultant solution was filtered with a 0.45 micron membrane filter and the solvent was removed at vacuum to obtain white solid. The solid was dried and weighed to obtain 4.15 g of a silver complex (yield = 93.04 %). Most of the silver complex was decomposed below 130 °C and the silver content was 18.79 wt% (TGA analysis).

### Example 28: Reaction of silver oxide with isopropylammonium bicarbonate

In a 50 mL Schlenk flask equipped with a stirrer, 2.97 g (24.51 mmol) of isopropylammonium bicarbonate (melting point: 53-54 °C) was dissolved in 10 mL of methanol and 1.0 g (4.31 mmol) of silver oxide was added. The reaction solution was initially a black slurry but it turned transparent as complex was produced. After 2 hours, a colorless, transparent solution was obtained. The resultant solution was filtered with a 0.45 micron membrane filter and the solvent was removed at vacuum to obtain white solid. The solid was dried and weighed to obtain 2.41 g of a silver complex (yield = 60.7 %). The silver complex had a melting point of 68-70 °C (DSC = 70.49 °C). Most of the silver complex was decomposed below 130 °C to leave metallic silver and the silver content was 38.58 wt% (TGA analysis).

### Example 29: Reaction of silver oxide with ammonium carbonate

In a 250 mL Schlenk flask equipped with a stirrer, 8.26 g (86 mmol) of ammonium carbonate and 15 g (0.25 mol) of isopropyl amine were dissolved in 50 mL of methanol and 10.0 g (43.1 mmol) of silver oxide was added. The reaction solution was initially a black slurry but it turned transparent as complex was produced. After 2 hours, a colorless, transparent solution was obtained. The resultant solution was filtered with a 0.45 micron membrane filter and the solvent was removed at vacuum to obtain white solid. The solid was dried and weighed to obtain 28.38 g of a silver complex (yield = 85.5 %). The silver complex had a melting point (DSC) of 63.38 °C. Most of the silver complex was decomposed below 130 °C to leave metallic silver and the silver content was 46.3 wt% (TGA analysis).

### Example 30: Reaction of silver carbonate with ammonium carbonate

In a 250 mL Schlenk flask equipped with a stirrer, 4.13 g (43 mmol) of ammonium carbonate and 15 g (0.25 mol) of isopropyl amine were dissolved in 50 mL of methanol and 11.88 g (43.1 mmol) of silver carbonate was added. The reaction solution was initially a yellow slurry but it turned transparent as complex was produced. After 6 hours, a colorless, transparent solution was obtained. The resultant solution was filtered with a 0.45 micron membrane filter and the solvent was removed at vacuum to obtain white solid. The solid was dried and weighed to obtain 26.71 g of a silver complex (yield = 85.9 %). Most of the silver complex was decomposed below 130 °C to leave metallic silver and the silver content was 47.8 wt% (TGA analysis).

### Example 31: Reaction of silver oxide with ammonium bicarbonate

In a 250 mL Schlenk flask equipped with a stirrer, 6.8 g (86 mmol) of ammonium bicarbonate and 15 g (0.25 mol) of isopropyl amine were dissolved in 50 mL of methanol and 10.0 g (43.1 mmol) of silver oxide was added. The reaction solution was initially a black slurry but it turned transparent as complex was produced. After 3 hours, a colorless, transparent solution was obtained. The resultant solution was filtered with a 0.45 micron membrane filter and the solvent was removed at vacuum to obtain white solid. The solid was dried and weighed to obtain 26.55 g of a silver complex (yield = 83.5 %). Most of the silver complex was decomposed below 130 °C to leave metallic silver and the silver content was 46.8 wt% (TGA analysis).

### Example 32: Reaction of silver carbonate with ammonium bicarbonate

In a 250 mL Schlenk flask equipped with a stirrer, 3.4 g (43 mmol) of ammonium bicarbonate and 15 g (0.25 mol) of isopropyl amine were dissolved in 50 mL of methanol and 11.88 g (43.1 mmol) of silver carbonate was added. The reaction solution was initially a yellow slurry but it turned transparent as complex was produced. After 6 hours, a colorless, transparent solution was obtained. The resultant solution was filtered with a 0.45 micron membrane filter and the solvent was removed at vacuum to obtain white solid. The solid was dried and weighed to obtain 26.20 g of a silver complex (yield = 86.2 %). Most of the silver complex was decomposed below 130 °C to leave metallic silver and the silver content was 48.2 wt% (TGA analysis).

### Example 33: Reaction of silver oxide with 2-methoxyethylammonium bicarbonate

In a 50 mL Schlenk flask equipped with a stirrer, 3.24 g (23.65 mmol) of 2-methoxyethylammonium bicarbonate (viscous liquid) was dissolved in 10 mL of methanol and 1.0 g (4.31 mmol) of silver oxide was added. The reaction solution was initially a black slurry but it turned transparent as complex was produced. After 2 hours, a yellow, transparent solution was obtained. The resultant solution was filtered with a 0.45 micron membrane filter and the solvent was removed at vacuum to obtain yellow, viscous liquid. The liquid was dried and weighed to obtain 3.01 g of a silver complex (yield = 70.75 %). Most of the silver complex was decomposed below 130 °C to leave metallic silver and the silver content was 31.08 wt% (TGA analysis).

### Example 34: Reaction of silver carbonate with 2-methoxyethylammonium bicarbonate

In a 50 mL Schlenk flask equipped with a stirrer, 3.78 g (27.54 mmol) of 2-methoxyethylammonium bicarbonate (viscous liquid) was dissolved in 10 mL of methanol and 1.0 g (3.60 mmol) of silver carbonate was added. The reaction solution was initially a yellow slurry but it turned transparent as complex was produced. After 2 hours, a yellow, transparent solution was obtained. The resultant solution was filtered with a 0.45 micron membrane filter and the solvent was removed at vacuum to obtain yellow, viscous liquid. The liquid was dried and weighed to obtain 3.28 g of a silver complex (yield = 68.61 %). Most of the silver complex was decomposed below 130 °C to leave metallic silver and the silver content was 23.78 wt% (TGA analysis).

### Example 35: Reaction of silver oxide with octylammonium bicarbonate

In a 50 mL Schlenk flask equipped with a stirrer, 3.07 g (24.73 mmol) of octylammonium bicarbonate (white solid) was dissolved in 10 mL of methanol and 1.0 g (4.31 mmol) of silver oxide was added. The reaction solution was initially a black slurry but it turned transparent as complex was produced. After 2 hours, a colorless, transparent solution was obtained. The resultant solution was filtered with a 0.45 micron membrane filter and the solvent was removed at vacuum to obtain white solid. The solid was dried and weighed to obtain 3.81 g of a silver complex (yield = 93.61 %). Most of the silver complex was decomposed below 130 °C to leave metallic silver and the silver content was 24.40 wt% (TGA analysis).

### Example 36: Reaction of silver oxide with isobutylammonium bicarbonate

In a 50 mL Schlenk flask equipped with a stirrer, 3.20 g (23.65 mmol) of isobutylammonium bicarbonate (white solid) was dissolved in 10 mL of methanol and 1.0 g (4.31 mmol) of silver oxide was added. The reaction solution was initially a black slurry but it turned transparent as complex was produced. After 2 hours, a colorless, transparent solution was obtained. The resultant solution was filtered with a 0.45 micron membrane filter and the solvent was removed at vacuum to obtain white solid. The solid was dried and weighed to obtain 3.21 g of a silver complex (yield = 76.42 %). Most of the silver complex was decomposed below 130 °C to leave metallic silver and the silver content was 28.97 wt% (TGA analysis).

### Example 37: Reaction of silver oxide with n-butylammonium bicarbonate

In a 50 mL Schlenk flask equipped with a stirrer, 3.20 g (23.65 mmol) of viscous n-butylammonium bicarbonate was dissolved in 10 mL of methanol and 1.0 g (4.31 mmol) of silver oxide was added. The reaction solution was initially a black slurry but it turned transparent as complex was produced. After 6 hours, a colorless, transparent solution was obtained. The resultant solution was filtered with a 0.45 micron membrane filter and the solvent was removed at vacuum to obtain white solid. The solid was dried and weighed to obtain 3.49 g of a silver complex (yield = 83.09 %). Most of the silver complex was decomposed below 130 °C to leave metallic silver and the silver content was 26.72 wt% (TGA analysis).

### Example 38: Reaction of silver oxide with morpholinium bicarbonate

In a 50 mL Schlenk flask equipped with a stirrer, 3.53 g (23.65 mmol) of morpholinium bicarbonate (white solid) was dissolved in 10 mL of methanol and 1.0 g (4.31 mmol) of silver oxide was added. The reaction solution was initially a black slurry but it turned transparent as complex was produced. After 2 hours, a yellow, transparent solution was obtained. The resultant solution was filtered with a 0.45 micron membrane filter and the solvent was removed at vacuum to obtain white solid. The solid was dried and weighed to obtain 3.16 g of a silver complex (yield = 69.75 %). Most of the silver complex was decomposed below 130 °C to leave metallic silver and the silver content was 29.49 wt% (TGA analysis).

### Example 39: Reaction of silver oxide with 2-ethylhexylammonium 2-ethylhexylcarbonate

In a 50 mL Schlenk flask equipped with a stirrer, 4.13 g (12.90 mmol) of 2-ethylhexylammonium 2-ethylhexylcarbonate (viscous liquid) was dissolved in 10 mL of tetrahydrofuran (THF) and 1.0 g (4.31 mmol) of silver oxide was added. The reaction solution was initially a black slurry but it turned transparent as complex was produced. After 2 hours, a yellow, transparent solution was obtained. The resultant solution was filtered with a 0.45 micron membrane filter and the solvent was removed at vacuum to obtain white solid. The solid was dried and weighed to obtain 4.05 g of a silver complex (yield = 78.84 %). Most of the silver complex was decomposed below 130 °C to leave metallic silver and the silver content was 22.96 wt% (TGA analysis).

### Example 40: Reaction of silver oxide with 2-ethylhexylammonium 2-ethylhexylcarbonate

In a 50 mL Schlenk flask equipped with a stirrer, 4.13 g (12.90 mmol) of 2-ethylhexylammonium 2-ethylhexylcarbonate (viscous liquid) was dissolved in 10 mL of ethyl acetate and 1.0 g (4.31 mmol) of silver oxide was added. The reaction solution was initially a black slurry but it turned transparent as complex was produced. After 2 hours, a colorless, transparent solution was obtained. The resultant solution was filtered with a 0.45 micron membrane filter and the solvent was removed at vacuum to obtain white solid. The solid was dried and weighed to obtain 3.96 g of a silver complex (yield = 77.19 %). Most of the silver complex was decomposed below 130 °C to leave metallic silver and the silver content was 23.48 wt% (TGA analysis).

### Example 41: Reaction of silver oxide with 2-ethylhexylammonium 2-ethylhexylcarbonate

Into a 50 mL Schlenk flask equipped with a stirrer were added 4.13 g (12.90 mmol) of 2-ethylhexylammonium 2-ethylhexylcarbonate (viscous liquid) and 1.0 g (4.31 mmol) of silver oxide. The reaction solution was initially a black slurry but it turned transparent as complex was produced. After 2 hours, a yellow, transparent solution was obtained. The resultant solution was filtered with a 0.45 micron membrane filter and the solvent was removed at vacuum to obtain white solid. The solid was dried and weighed to obtain 3.96 g of a silver complex (yield = 77.19 %). Most of the silver complex was decomposed below 130 °C to leave metallic silver and the silver content was 23.48 wt% (TGA analysis).

### Example 42: Reaction of silver oxide with aminoethylammonium aminoethylcarbonate

In a 50 mL Schlenk flask equipped with a stirrer, 2.35 g (12.90 mmol) of aminoethylammonium aminoethylcarbonate (white solid) was dissolved in 10 mL of methanol and 1.0 g (4.31 mmol) of silver oxide was added. The reaction solution was initially a black slurry but it turned transparent as complex was produced. After 2 hours, a colorless, transparent solution was obtained. The resultant solution was filtered with a 0.45 micron membrane filter and the solvent was removed at vacuum to obtain black, viscous liquid. The liquid was dried and weighed to obtain 2.42 g of a silver complex (yield = 72.23 %). Most of the silver complex was decomposed below 130 °C to leave metallic silver and the silver content was 38.45 wt% (TGA analysis).

### Example 43: Reaction of silver oxide with 2-ethylhexylammonium 2-ethylhexylcarbonate and aminoethylammonium aminoethylcarbonate

In a 50 mL Schlenk flask equipped with a stirrer, 3.87 g (12.9 mmol) of a 6:1 (molar ratio) mixture of 2-ethylhexylammonium 2-ethylhexylcarbonate and aminoethylammonium aminoethylcarbonate was dissolved in 10 mL of methanol and 1.0 g (4.31 mmol) of silver oxide was added. The reaction solution was initially a black slurry but it turned transparent as complex was produced. After 2 hours of reaction, a colorless, transparent solution was obtained. The resultant solution was filtered with a 0.45 micron membrane filter and the solvent was removed at vacuum to obtain orange, viscous liquid. The liquid was dried and weighed to obtain 3.05 g of a silver complex (yield = 78.85 %). Most of the silver complex was decomposed below 130 °C to leave metallic silver. The silver content was 30.41 wt% (TGA analysis).

### Example 44: Reaction of silver sulfate with 2-ethylhexylammonium 2-ethylhexylcarbonate

In a 50 mL Schlenk flask equipped with a stirrer, 3.07 g (9.60 mmol) of 2-ethylhexylammonium 2-ethylhexylcarbonate (viscous liquid) was dissolved in 10 mL of methanol and 1.0 g (3.2 mmol) of silver sulfate was added. The reaction solution was initially a white slurry but it turned transparent as complex was produced. After 2 hours, a completely transparent solution was obtained. The resultant solution was filtered with a 0.45 micron membrane filter and the solvent was removed at vacuum to obtain white solid. The solid was recrystallized in ethyl acetate, dried and weighed to obtain 3.55 g of a silver complex (yield = 87.2 %). Most of the silver complex was decomposed below 130 °C to leave metallic silver and the silver content was 19.52 wt% (TGA analysis).

### Example 45: Reaction of silver nitrate with 2-ethylhexylammonium 2-ethylhexylcarbonate

In a 50 mL Schlenk flask equipped with a stirrer, 2.84 g (8.86 mmol) of 2-ethylhexylammonium 2-ethylhexylcarbonate (viscous liquid) was dissolved in 10 mL of methanol and 1.0 g (5.9 mmol) of silver nitrate was added. The reaction solution was initially a white slurry but it turned transparent as complex was produced. After 2 hours, a completely transparent solution was obtained. The resultant solution was filtered with a 0.45 micron membrane filter and the solvent was removed at vacuum to obtain white solid. The solid was recrystallized in ethyl acetate, dried and weighed to obtain 3.12 g of a silver complex (yield = 81.34 %). Most of the silver complex was decomposed below 130 °C to leave metallic silver and the silver content was 19.88 wt% (TGA analysis).

### Example 46: Reaction of silver cyanide with 2-ethylhexylammonium 2-ethylhexylcarbonate

In a 50 mL Schlenk flask equipped with a stirrer, 3.59 g (11.20 mmol) of 2-ethylhexylammonium 2-ethylhexylcarbonate (viscous liquid) was dissolved in 10 mL of methanol and 1.0 g (7.5 mmol) of silver cyanide was added. The reaction solution was initially a white slurry but it turned transparent as complex was produced. After 2 hours, a completely transparent solution was obtained. The resultant solution was filtered with a 0.45 micron membrane filter and the solvent was removed at vacuum to obtain white solid. The solid was recrystallized in ethyl acetate, dried and weighed to obtain 3.93 g of a silver complex (yield = 85.62 %). Most of the silver complex was decomposed below 130 °C to leave metallic silver and the silver content was 20.37 wt% (TGA analysis).

From the silver contents of the prepared compounds, m values were calculated. They are given in Table 1 below.

**Table 1**

| Example No. | m value | Example No. | m value | Example No. | m value |
|---|---|---|---|---|---|
| 1 | 1.3 | 17 | 1.0 | 33 | 1.8 |
| 2 | 1.1 | 18 | 0.9 | 34 | 2.5 |
| 3 | 1.1 | 19 | 0.7 | 35 | 1.8 |
| 4 | 1.1 | 20 | 1.3 | 36 | 2.0 |
| 5 | 1.2 | 21 | 1.2 | 37 | 2.2 |
| 6 | 1.2 | 22 | 1.1 | 38 | 1.6 |
| 7 | 1.6 | 23 | 1.2 | 39 | 1.1 |
| 8 | 1.0 | 24 | 2.1 | 40 | 1.1 |
| 9 | 1.1 | 25 | 1.0 | 41 | 1.1 |
| 10 | 1.4 | 26 | 1.3 | 42 | 1.0 |
| 11 | 1.1 | 27 | 1.5 | 43 | 0.8 |
| 12 | 1.1 | 28 | 1.6 | 44 | 1.4 |
| 13 | 1.9 | 29 | 1.3 | 45 | 1.4 |
| 14 | 1.6 | 30 | 1.2 | 46 | 1.3 |
| 15 | 1.0 | 31 | 1.6 | - | - |
| 16 | 1.0 | 32 | 1.5 | - | - |

### Example 47

4 g of the silver complex prepared in Example 1 was dissolved in 5 g of butyl alcohol. After adjusting the viscosity to 500 cps, pattering was performed on a coated paper (ITP20HPG or ITP20SPH; InkTec) on a silk screen patterned to 320 meshes using a stainless steel (SUS) wire cloth. After heat treatment at 100 °C for 5 minutes and then at 130 °C for 10 minutes, a metal pattern having a conductivity of 400-500 mΩ/□ was obtained.

### Example 48

4 g of the silver complex prepared in Example 1 was dissolved in 10 g of isopropyl alcohol. After adjusting the viscosity to 13 cps, pattering was performed on a PET film for one time using an ink-jet printer. After heat treatment at 80 °C for 5 minutes and then at 130 °C for 10 minutes, a metal pattern having a conductivity of 200-300 mΩ/□ was obtained.

### Example 49

4 g of the silver complex prepared in Example 23 was dissolved in 5 g of 2-hexyl alcohol. After adjusting the viscosity to 500 cps, pattering was performed on a coated paper (ITP20HPG or ITP20SPH; InkTec) on a 320-mesh patterned silk screen. After heat treatment at 100 °C for 5 minutes and then at 130 °C for 10 minutes, a metal pattern having a conductivity of 400-500 mΩ/□ was obtained.

### Example 50

4 g of the silver complex prepared in Example 24 was dissolved in 10 g of butyl alcohol. After adjusting the viscosity to 13 cps, pattering was performed on a PET film for one time using an ink-jet printer. After heat treatment at 80 °C for 5 minutes and then at 130 °C for 10 minutes, a metal pattern having a conductivity of 200-300 mΩ/□ was obtained.

### Industrial Applicability

The present invention provides a useful organic silver complex by reacting the silver compound represented by the formula 2 with the ammonium carbamate compound or the ammonium carbonate compound represented by the formula 3, 4 or 5.

As the TGA analysis shows, the organic silver complex of the present invention is decomposed at a very low temperature to give pure metal film or powder. So, it can be processed into a variety of metallic silver films or formed into an ultrathin film by deposition under high vacuum. Thus, it can be used in plating, medicine, photography, electricity and electronics, fibers, detergents, household appliances, organics and polymer synthesis as catalyst or may be used in preparation of silver powder, paste and nanoparticle. Particularly, it may be utilized in low-resistance metal wirings, printed circuit boards (PCB), flexible printed circuit boards (FPC), antennas for radio frequency identification (RFID) tags, plasma display panels (PDP), liquid crystal displays (TFT-LCD), organic light emitting diodes (OLED), flexible displays, organic thin-film transistors (OTFT), electrodes, etc. as precursor material for metal patterning by chemical vapor deposition (CVD), plasma vapor deposition, sputtering, electroplating, lithography, electron beam, laser, etc. In addition, the organic silver complex solution of the present invention may be spin coated, roll coated, spray coated, dip coated, flow coated, ink-jet printed, offset printed, screen printed, gravure printed or flexo printed on such a substrate as glass, silicon wafer and polymer film like polyester or polyimide, paper, etc. and reduced, oxidized or heat-treated to form a metal or metal oxide pattern.

## Claims

1. A silver complex obtained by reacting at least one silver compound represented by the formula 2 below:
AgₙX (2)
where n is an integer from 1 to 4 and X is a substituent selected from the group consisting of oxygen, sulfur, halogen, cyano, cyanate, carbonate, nitrate, nitrite, sulfate, phosphate, thiocyanate, chlorate, perchlorate, tetrafluoroborate, acetylacetonate and carboxylate; with at least one ammonium carbamate compound selected from ethylammonium ethylcarbamate, isopropylammonium isopropylcarbamate, n-butylammonium n-butylcarbamate, isobutylammonium isobutylcarbamate, t-butylammonium t-butylcarbamate, 2-ethylhexylammonium 2-ethylhexylcarbamate, octadecylammonium octadecylcarbamate, 2-methoxyethylammonium 2-methoxyethylcarbamate, 2-cyanoethylammonium 2-cyanoethylcarbamate, dibutylammonium dibutylcarbamate, dioctadecylammonium dioctadecylcarbamate, methyldecylammonium methyldecylcarbamate, hexamethyleneiminium hexamethyleneiminecarbamate, morpholinium morpholinecarbamate, pyridinium ethylhexylcarbamate, triethylenediaminium isopropylbicarbamate, benzylammonium benzylcarbamate and triethoxysilylpropylammonium triethoxysilylpropylcarbamate; and/or at least one ammonium carbonate compound selected from ethylammonium ethylcarbonate, isopropylammonium isopropylcarbonate, isopropylammonium bicarbonate, n-butylammonium n-butylcarbonate, isobutylammonium isobutylcarbonate, t-butylammonium t-butylcarbonate, t-butylammonium bicarbonate, 2-ethylhexylammonium 2-ethylhexylcarbonate, 2-ethylhexylammonium bicarbonate, 2-methoxyethylammonium 2-methoxyethylcarbonate, 2-methoxyethylammonium bicarbonate, 2-cyanoethylammonium 2-cyanoethylcarbonate, 2-cyanoethylammonium bicarbonate, octadecylammonium octadecylcarbonate, dibutylammonium dibutylcarbonate, dioctadecylammonium dioctadecylcarbonate, dioctadecylammonium bicarbonate, methyldecylammonium methyldecylcarbonate, hexamethyleneiminium hexamethyleneiminecarbonate, morpholinium morpholinecarbonate, benzylammonium benzylcarbonate, triethoxysilylpropylammonium triethoxysilylpropylcarbonate, pyridinium bicarbonate, triethylenediaminium isopropylcarbonate and triethylenediaminium bicarbonate.

2. The silver complex of Claim 1, which is represented by the formula 1 below:
Ag[A]ₘ (1)
where A is an ammonium carbamate compound or an ammonium carbonate compound as defined in claim 1 and 0.7 ≤ m ≤ 2.5.

3. The silver complex of Claim 1, wherein the silver compound represented by the formula 2 is at least one selected from silver oxide, thiocyanate, silver cyanide, silver cyanate, silver carbonate, silver nitrate, silver nitrite, silver sulfate, silver phosphate, silver perchlorate, silver tetrafluoroborate, silver acetylacetonate, silver acetate, silver lactate and silver oxalate.

4. The silver complex of Claim 1, wherein the silver compound represented by the formula 2 is silver oxide, silver carbonate or a mixture thereof.

5. The silver complex of Claim 1, which is obtained by reacting
- silver oxide with 2-ethylhexylammonium 2-ethylhexylcarbamate,
- silver oxide with isopropylammonium isopropylcarbamate,
- silver oxide with n-butylammonium n-butylcarbamate,
- silver oxide with isobutylammonium isobutylcarbamate,
- silver oxide with t-butylammonium t-butylcarbamate,
- silver carbonate with 2-ethylhexylammonium 2-ethylhexylcarbamate,
- silver oxide with 2-methoxyethylammonium 2-methoxyethylcarbamate,
- silver oxide with 2-cyanoethylammonium 2-cyanoethylcarbamate,
- silver oxide with morpholinium morpholinecarbamate,
- silver oxide with hexamethyleneiminium hexamethyleneiminecarbamate,
- silver sulfate with 2-ethylhexylammonium 2-ethylhexylcarbamate,
- silver nitrate with 2-ethylhexylammonium 2-ethylhexylcarbamate,
- silver cyanide with 2-ethylhexylammonium 2-ethylhexylcarbamate,
- silver oxide with 2-ethylhexylammonium 2-ethylhexylcarbonate,
- silver oxide with 2-ethylhexylammonium bicarbonate,
- silver oxide with isopropylammonium isopropylcarbonate,
- silver carbonate with isopropylammonium isopropylcarbonate,
- silver carbonate with 2-ethylhexylammonium 2-ethylhexylcarbonate,
- silver oxide with isopropylammonium bicarbonate,
- silver oxide with 2-methoxyethylammonium bicarbonate,
- silver carbonate with 2-methoxyethylammonium bicarbonate,
- silver sulfate with 2-ethylhexylammonium 2-ethylhexylcarbonate,
- silver nitrate with 2-ethylhexylammonium 2-ethylhexylcarbonate, or
- silver cyanide with 2-ethylhexylammonium 2-ethylhexylcarbonate.

6. A method for forming a metal film or a metal oxide film by forming a thin film using the silver complex of any of Claims 1 to 4 and performing oxidation, reduction, heat treatment, chemical vapor deposition, plasma vapor deposition, sputtering, electroplating, lithography, IR, electron beam or laser treatment.

7. The method of Claim 6, wherein the thin film is formed by coating on a substrate.

8. The method of Claim 7, wherein the substrate is selected from glass, silicon, polyester, polyimide and paper.

9. The method of Claim 6, wherein the heat treatment is performed using air, nitrogen, argon, hydrogen or a mixture gas thereof.

10. The method of Claim 7, wherein the coating is performed by spin coating, roll coating, spray coating, dip coating or flow coating.

11. The method of Claim 7, wherein the coating is performed by a printing method selected from ink-jet printing, offset printing, screen printing, gravure printing and flexo printing.

12. The method of Claim 7, wherein the coating is performed using a silver complex solution prepared by dissolving the silver complex in a solvent selected from alcohol, glycol, acetate, ether, ketone, aliphatic hydrocarbon, aromatic hydrocarbon and halogenated hydrocarbon.

13. The method of Claim 12, wherein the solvent is at least one selected from methanol, ethanol, isopropanol, butanol, ethylene glycol, glycerine, ethyl acetate, butyl acetate, carbitol acetate, diethyl ether, tetrahydrofuran, dioxane, methyl ethyl ketone, acetone, hexane, heptane, benzene, toluene, chloroform, methylene chloride and carbon tetrachloride.

14. A silver complex solution prepared by dissolving the silver complex of any of Claims 1 to 4 in a solvent selected from alcohol, glycol, acetate, ether, ketone, aliphatic hydrocarbon, aromatic hydrocarbon and halogenated hydrocarbon.

15. The silver complex solution of Claim 14, wherein the solvent is at least one selected from methanol, ethanol, isopropanol, butanol, ethylene glycol, glycerine, ethyl acetate, butyl acetate, carbitol acetate, diethyl ether, tetrahydrofuran, dioxane, methyl ethyl ketone, acetone, hexane, heptane, benzene, toluene, chloroform, methylene chloride and carbon tetrachloride.

16. A method for preparing a silver complex by reacting at least one silver compound represented by the formula 2 below at room temperature in the presence of a solvent:
AgₙX (2)
where n is an integer from 1 to 4 and X is a substituent selected from the group consisting of oxygen, sulfur, halogen, cyano, cyanate, carbonate, nitrate, nitrite, sulfate, phosphate, thiocyanate, chlorate, perchlorate, tetrafluoroborate, acetylacetonate and carboxylate; with at least one ammonium carbamate compound selected from ethylammonium ethylcarbamate, isopropylammonium isopropylcarbamate, n-butylammonium n-butylcarbamate, isobutylammonium isobutylcarbamate, t-butylammonium t-butylcarbamate, 2-ethylhexylammonium 2-ethylhexylcarbamate, octadecylammonium octadecylcarbamate, 2-methoxyethylammonium 2-methoxyethylcarbamate, 2-cyanoethylammonium 2-cyanoethylcarbamate, dibutylammonium dibutylcarbamate, dioctadecylammonium dioctadecylcarbamate, methyldecylammonium methyldecylcarbamate, hexamethyleneiminium hexamethyleneiminecarbamate, morpholinium morpholinecarbamate, pyridinium ethylhexylcarbamate, triethylenediaminium isopropylbicarbamate, benzylammonium benzylcarbamate and triethoxysilylpropylammonium triethoxysilylpropylcarbamate; and/or at least one ammonium carbonate compound selected from ethylammonium ethylcarbonate, isopropylammonium isopropylcarbonate, isopropylammonium bicarbonate, n-butylammonium n-butylcarbonate, isobutylammonium isobutylcarbonate, t-butylammonium t-butylcarbonate, t-butylammonium bicarbonate, 2-ethylhexylammonium 2-ethylhexylcarbonate, 2-ethylhexylammonium bicarbonate, 2-methoxyethylammonium 2-methoxyethylcarbonate, 2-methoxyethylammonium bicarbonate, 2-cyanoethylammonium 2-cyanoethylcarbonate, 2-cyanoethylammonium bicarbonate, octadecylammonium octadecylcarbonate, dibutylammonium dibutylcarbonate, dioctadecylammonium dioctadecylcarbonate, dioctadecylammonium bicarbonate, methyldecylammonium methyldecylcarbonate, hexamethyleneiminium hexamethyleneiminecarbonate, morpholinium morpholinecarbonate, benzylammonium benzylcarbonate, triethoxysilylpropylammonium triethoxysilylpropylcarbonate, pyridinium bicarbonate, triethylenediaminium isopropylcarbonate and triethylenediaminium bicarbonate.

17. The method of Claim 16, wherein the silver complex is represented by the formula 1 below:
Ag[A]ₘ (1)
where A is an ammonium carbamate compound or an ammonium carbonate compound as defined in claim 16 and 0.7 ≤ m ≤ 2.5.

18. The method of Claim 16, wherein the solvent is at least one selected from methanol, ethanol, isopropanol, butanol, ethylene glycol, glycerine, ethyl acetate, butyl acetate, carbitol acetate, diethyl ether, tetrahydrofuran, dioxane, methyl ethyl ketone, acetone, hexane, heptane, benzene, toluene, chloroform, methylene chloride and carbon tetrachloride.

19. A silver complex which is obtained by reacting
- silver oxide with n-propylammonium n-propylcarbamate,
- silver oxide with 2-hydroxyethylammonium 2-hydroxyethylcarbamate,
- silver oxide with aminoethylammonium aminoethylcarbamate,
- silver oxide with 2-ethylhexylammonium 2-ethylhexylcarbamate and aminoethylammonium aminoethylcarbamate,
- silver oxide with octylammonium bicarbonate,
- silver oxide with isobutylammonium bicarbonate,
- silver oxide with n-butylammonium bicarbonate,
- silver oxide with morpholinium bicarbonate,
- silver oxide with aminoethylammonium aminoethylcarbonate,
- silver oxide with 2-ethylhexylammonium 2-ethylhexylcarbonate and aminoethylammonium aminoethylcarbonate,
- silver oxide with ammonium carbamate,
- silver carbonate with ammonium carbamate,
- silver oxide with ammonium carbonate,
- silver carbonate with ammonium carbonate,
- silver oxide with ammonium bicarbonate, or
- silver carbonate with ammonium bicarbonate.

## Patentansprüche

1. Silberkomplex, erhalten durch Umsetzen wenigstens einer Silberverbindung, dargestellt durch die Formel 2:
AgₙX (2)
wobei n eine ganze Zahl von 1 bis 4 ist und X ein Substituent ist, ausgewählt aus der Gruppe, bestehend aus Sauerstoff, Schwefel, Halogen, Cyano, Cyanat, Carbonat, Nitrat, Nitrit, Sulfat, Phosphat, Thiocyanat, Chlorat, Perchlorat, Tetrafluorborat, Acetylacetonat und Carboxylat;
mit wenigstens einer Ammoniumcarbamatverbindung, ausgewählt aus Ethylammoniumethylcarbamat, Isopropylammoniumisopropylcarbamat, n-Butylammonium-n-butylcarbamat, Isobutylammoniumisobutylcarbamat, t-Butylammonium-t-butylcarbamat, 2-Ethylhexylammonium-2-ethylhexylcarbamat, Octadecylammoniumoctadecylcarbamat, 2-Methoxyethylammonium-2-methoxyethylcarbamat, 2-Cyanoethylammonium-2-cyanoethylcarbamat, Dibutylammoniumdibutylcarbamat, Dioctadecylammoniumdioctadecylcarbamat, Methyldecylammoniummethyldecylcarbamat, Hexamethyleniminiumhexamethylenimincarbamat, Morpholiniummorpholincarbamat, Pyridiniumethylhexylcarbamat, Triethylendiaminiumisopropylbicarbamat, Benzylammoniumbenzylcarbamat und Triethoxysilylpropylammoniumtriethoxysilylpropylcarbamat; und/oder
wenigstens einer Ammoniumcarbonatverbindung, ausgewählt aus Ethylammoniumethylcarbonat, Isopropylammoniumisopropylcarbonat, Isopropylammoniumbicarbonat, n-Butylammonium-n-butylcarbonat, Isobutylammoniumisobutylcarbonat, t-Butylammonium-t-butylcarbonat, t-Butylammoniumbicarbonat, 2-Ethylhexylammonium-2-ethylhexylcarbonat, 2-Ethylhexylammoniumbicarbonat, 2-Methoxyethylammonium-2-methoxyethylcarbonat, 2-Methoxyethylammoniumbicarbonat, 2-Cyanoethylammonium-2-cyanoethylcarbonat, 2-Cyanoethylammoniumbicarbonat, Octadecylammoniumoctadecylcarbonat, Dibutylammoniumdibutylcarbonat, Dioctadecylammoniumdioctadecylcarbonat, Dioctadecylammoniumbicarbonat, Methyldecylammoniummethyldecylcarbonat, Hexamethyleniminiumhexamethylenimincarbonat, Morpholiniummorpholincarbonat, Benzylammoniumbenzylcarbonat, Triethoxysilylpropylammoniumtriethoxysilylpropylcarbonat, Pyridiniumbicarbonat, Triethylendiaminiumisopropylcarbonat und Triethylendiaminiumbicarbonat.

2. Silberkomplex gemäß Anspruch 1, der durch die folgende Formel 1 dargestellt wird:
Ag[A]ₘ (1)
worin A eine Ammoniumcarbamatverbindung oder eine Ammoniumcarbonatverbindung, wie in Anspruch 1 definiert, ist, und 0,7 ≤ m ≤ 2,5.

3. Silberkomplex gemäß Anspruch 1, wobei die durch die Formel 2 dargestellte Silberverbindung wenigstens eine ist, ausgewählt aus Silberoxid, Thiocyanat, Silbercyanid, Silbercyanat, Silbercarbonat, Silbernitrat, Silbernitrit, Silbersulfat, Silberphosphat, Silberperchlorat, Silbertetrafluorborat, Silberacetylacetonat, Silberacetat, Silberlactat und Silberoxalat.

4. Silberkomplex gemäß Anspruch 1, wobei die durch die Formel 2 dargestellte Silberverbindung Silberoxid, Silbercarbonat oder ein Gemisch davon ist.

5. Silberkomplex gemäß Anspruch 1, erhalten durch Umsetzen von
- Silberoxid mit 2-Ethylhexylammonium-2-ethylhexylcarbamat,
- Silberoxid mit Isopropylammoniumisopropylcarbamat,
- Silberoxid mit n-Butylammonium-n-butylcarbamat,
- Silberoxid mit Isobutylammoniumisobutylcarbamat,
- Silberoxid mit t-Butylammonium-t-butylcarbamat,
- Silbercarbonat mit 2-Ethylhexylammonium-2-ethylhexylcarbamat,
- Silberoxid mit 2-Methoxyethylammonium-2-methoxyethylcarbamat,
- Silberoxid mit 2-Cyanoethylammonium-2-cyanoethylcarbamat,
- Silberoxid mit Morpholiniummorpholincarbamat,
- Silberoxid mit Hexamethyleniminiumhexamethylenimincarbamat,
- Silbersulfat mit 2-Ethylhexylammonium-2-ethylhexylcarbamat,
- Silbernitrat mit 2-Ethylhexylammonium-2-ethylhexylcarbamat,
- Silbercyanid mit 2-Ethylhexylammonium-2-ethylhexylcarbamat,
- Silberoxid mit 2-Ethylhexylammonium-2-ethylhexylcarbonat,
- Silberoxid mit 2-Ethylhexylammoniumbicarbonat,
- Silberoxid mit Isopropylammoniumisopropylcarbonat,
- Silbercarbonat mit Isopropylammoniumisopropylcarbonat,
- Silbercarbonat mit 2-Ethylhexylammonium-2-ethylhexylcarbonat,
- Silberoxid mit Isopropylammoniumbicarbonat,
- Silberoxid mit 2-Methoxyethylammoniumbicarbonat,
- Silbercarbonat mit 2-Methoxyethylammoniumbicarbonat,
- Silbersulfat mit 2-Ethylhexylammonium-2-ethylhexylcarbonat,
- Silbernitrat mit 2-Ethylhexylammonium-2-ethylhexylcarbonat oder
- Silbercyanid mit 2-Ethylhexylammonium-2-ethylhexylcarbonat.

6. Verfahren zur Bildung eines Metallfilms oder eines Metalloxidfilms durch Ausbilden eines dünnen Films unter Verwendung des Silberkomplexes gemäß einem der Ansprüche 1 bis 4 und Ausführen von Oxidation, Reduktion, Wärmebehandlung, chemischer Dampfabscheidung, Plasmadampfabscheidung, Sputtern, Galvanisieren, Lithographie, IR, Elektronenstrahl- oder Laserbehandlung.

7. Verfahren gemäß Anspruch 6, wobei der dünne Film durch Auftragen auf ein Substrat gebildet wird.

8. Verfahren gemäß Anspruch 7, wobei das Substrat aus Glas, Silicium, Polyester, Polyimid und Papier ausgewählt ist.

9. Verfahren gemäß Anspruch 6, wobei die Wärmebehandlung unter Verwendung von Luft, Stickstoff, Argon, Wasserstoff oder einem Mischgas davon ausgeführt wird.

10. Verfahren gemäß Anspruch 7, wobei das Auftragen durch Rotationsbeschichtung, Walzenbeschichtung, Sprühbeschichtung, Tauchbeschichtung oder Fluten ausgeführt wird.

11. Verfahren gemäß Anspruch 7, wobei das Auftragen durch ein Druckverfahren, ausgewählt aus Tintenstrahldruck, Offsetdruck, Siebdruck, Tiefdruck und Flexodruck, ausgeführt wird.

12. Verfahren gemäß Anspruch 7, wobei das Auftragen unter Verwendung einer Silberkomplexlösung, hergestellt durch Auflösen des Silberkomplexes in einem Lösungsmittel, ausgewählt aus Alkohol, Glycol, Acetat, Ether, Keton, aliphatischem Kohlenwasserstoff, aromatischem Kohlenwasserstoff und halogeniertem Kohlenwasserstoff, ausgeführt wird.

13. Verfahren gemäß Anspruch 12, wobei das Lösungsmittel wenigstens eines ist, ausgewählt aus Methanol, Ethanol, Isopropanol, Butanol, Ethylenglycol, Glycerin, Ethylacetat, Butylacetat, Carbitolacetat, Diethylether, Tetrahydrofuran, Dioxan, Methylethylketon, Aceton, Hexan, Heptan, Benzol, Toluol, Chloroform, Methylenchlorid und Kohlenstofftetrachlorid.

14. Silberkomplexlösung, hergestellt durch Auflösen des Silberkomplexes gemäß einem der Ansprüche 1 bis 4 in einem Lösungsmittel, ausgewählt aus Alkohol, Glycol, Acetat, Ether, Keton, aliphatischem Kohlenwasserstoff, aromatischem Kohlenwasserstoff und halogeniertem Kohlenwasserstoff.

15. Silberkomplexlösung gemäß Anspruch 14, wobei das Lösungsmittel wenigstens eines ist, ausgewählt aus Methanol, Ethanol, Isopropanol, Butanol, Ethylenglycol, Glycerin, Ethylacetat, Butylacetat, Carbitolacetat, Diethylether, Tetrahydrofuran, Dioxan, Methylethylketon, Aceton, Hexan, Heptan, Benzol, Toluol, Chloroform, Methylenchlorid und Kohlenstofftetrachlorid.

16. Verfahren zur Herstellung eines Silberkomplexes durch Umsetzen wenigstens einer Silberverbindung, dargestellt durch die folgende Formel 2 bei Raumtemperatur in Gegenwart eines Lösungsmittels
AgₙX (2)
wobei n eine ganze Zahl von 1 bis 4 ist und X ein Substituent ist, ausgewählt aus der Gruppe, bestehend aus Sauerstoff, Schwefel, Halogen, Cyano, Cyanat, Carbonat, Nitrat, Nitrit, Sulfat, Phosphat, Thiocyanat, Chlorat, Perchlorat, Tetrafluorborat, Acetylacetonat und Carboxylat;
mit wenigstens einer Ammoniumcarbamatverbindung, ausgewählt aus Ethylammoniumethylcarbamat, Isopropylammoniumisopropylcarbamat, n-Butylammonium-n-butylcarbamat, Isobutylammoniumisobutylcarbamat, t-Butylammonium-t-butylcarbamat, 2-Ethylhexylammonium-2-ethylhexylcarbamat, Octadecylammoniumoctadecylcarbamat, 2-Methoxyethylammonium-2-methoxyethylcarbamat, 2-Cyanoethylammonium-2-cyanoethylcarbamat, Dibutylammoniumdibutylcarbamat, Dioctadecylammoniumdioctadecylcarbamat, Methyldecylammoniummethyldecylcarbamat, Hexamethyleniminiumhexamethylenimincarbamat, Morpholiniummorpholincarbamat, Pyridiniumethylhexylcarbamat, Triethylendiaminiumisopropylbicarbamat, Benzylammoniumbenzylcarbamat und Triethoxysilylpropylammoniumtriethoxysilylpropylcarbamat; und/oder
wenigstens einer Ammoniumcarbonatverbindung, ausgewählt aus Ethylammoniumethylcarbonat, Isopropylammoniumisopropylcarbonat, Isopropylammoniumbicarbonat, n-Butylammonium-n-butylcarbonat, Isobutylammoniumisobutylcarbonat, t-Butylammonium-t-butylcarbonat, t-Butylammoniumbicarbonat, 2-Ethylhexylammonium-2-ethylhexylcarbonat, 2-Ethylhexylammoniumbicarbonat, 2-Methoxyethylammonium-2-methoxyethylcarbonat, 2-Methoxyethylammoniumbicarbonat, 2-Cyanoethylammonium-2-cyanoethylcarbonat, 2-Cyanoethylammoniumbicarbonat, Octadecylammoniumoctadecylcarbonat, Dibutylammoniumdibutylcarbonat, Droctadecylammoniumdioctadecylcarbonat, Dioctadecylammoniumbicarbonat, Methyldecylammoniummethyldecylcarbonat, Hexamethyleniminiumhexamethylenimincarbonat, Morpholiniummorpholincarbonat, Benzylammoniumbenzylcarbonat, Triethoxysilylpropylammoniumtriethoxysilylpropylcarbonat, Pyridiniumbicarbonat, Triethylendiaminiumisopropylcarbonat und Triethylendiaminiumbicarbonat.

17. Verfahren gemäß Anspruch 16, wobei der Silberkomplex durch die folgende Formel 1 dargestellt wird:
Ag[A]ₘ (1)
worin A eine Ammoniumcarbamatverbindung oder eine Ammoniumcarbonatverbindung, wie in Anspruch 16 definiert, ist, und 0,7 ≤ m ≤ 2,5.

18. Verfahren gemäß Anspruch 16, wobei das Lösungsmittel wenigstens eines ist, ausgewählt aus Methanol, Ethanol, Isopropanol, Butanol, Ethylenglycol, Glycerin, Ethylacetat, Butylacetat, Carbitolacetat, Diethylether, Tetrahydrofuran, Dioxan, Methylethylketon, Aceton, Hexan, Heptan, Benzol, Toluol, Chloroform, Methylenchlorid und Kohlenstofftetrachlorid.

19. Silberkomplex, erhalten durch Umsetzen von
- Silberoxid mit n-Propylammonium-n-propylcarbamat,
- Silberoxid mit 2-Hydroxyethylammonium-2-hydroxyethylcarbamat,
- Silberoxid mit Aminoethylammoniumaminoethylcarbamat,
- Silberoxid mit 2-Ethylhexylammonium-2-ethylhexylcarbamat und Aminoethylammoniumaminoethylcarbamat,
- Silberoxid mit Octylammoniumbicarbonat,
- Silberoxid mit Isobutylammoniumbicarbonat,
- Silberoxid mit n-Butylammoniumbicarbonat,
- Silberoxid mit Morpholiniumbicarbonat,
- Silberoxid mit Aminoethylammoniumaminoethylcarbonat,
- Silberoxid mit 2-Ethylhexylammonium-2-ethylhexylcarbonat und Aminoethylammoniumaminoethylcarbonat,
- Silberoxid mit Ammoniumcarbamat,
- Silbercarbonat mit Ammoniumcarbamat,
- Silberoxid mit Ammoniumcarbonat,
- Silbercarbonat mit Ammoniumcarbonat,
- Silberoxid mit Ammoniumbicarbonat oder
- Silbercarbonat mit Ammoniumbicarbonat.

## Revendications

1. Complexe d'argent obtenu par réaction d'au moins un composé d'argent représenté par la formule 2 ci-dessous :
AgₙX (2)
dans laquelle n est un nombre entier de 1 à 4 et X est un substituant choisi dans le groupe constitué de l'oxygène, du soufre, d'un halogène, d'un groupe cyano, cyanate, carbonate, nitrate, nitrite, sulfate, phosphate, thiocyanate, chlorate, perchlorate, tétrafluoroborate, acétylacétonate et carboxylate ;
avec au moins un composé de carbamate d'ammonium choisi parmi l'éthylcarbamate d'éthyl-ammonium, l'isopropylcarbamate d'isopropyl-ammonium, le n-butylcarbamate de n-butyl-ammonium, l'isobutylcarbamate d'isobutyl-ammonium, le t-butylcarbamate de t-butyl-ammonium, le 2-éthylhexylcarbamate de 2-éthylhexyl-ammonium, l'octadécylcarbamate d'octadécyl-ammonium, le 2-méthoxyéthylcarbamate de 2-méthoxyéthyl-ammonium, le 2-cyanoéthylcarbamate de 2-cyanoéthyl-ammonium, le dibutylcarbamate de dibutyl-ammonium, le dioctadécylcarbamate de dioctadécyl-ammonium, le méthyldécylcarbamate de méthyldécyl-ammonium, l'hexaméthylèneiminecarbamate d'hexaméthylèneiminium, le morpholinecarbamate de morpholinium, l'éthylhexylcarbamate de pyridinium, l'isopropylbicarbamate de triéthylènediaminium, le benzylcarbamate de benzyl-ammonium et le triéthoxysilylpropylcarbamate de triéthoxysilylpropyl-ammonium ; et/ou
au moins un composé de carbonate d'ammonium choisi parmi l'éthylcarbonate d'éthyl-ammonium, l'isopropylcarbonate d'isopropyl-ammonium, le bicarbonate d'isopropyl-ammonium, le n-butylcarbonate de n-butyl-ammonium, l'isobutylcarbonate d'isobutylammonium, le t-butylcarbonate de t-butyl-ammonium, le bicarbonate de t-butyl-ammonium, le 2-éthylhexylcarbonate de 2-éthylhexyl-ammonium, le bicarbonate de 2-éthylhexyl-ammonium, le 2-méthoxyéthylcarbonate de 2-méthoxyéthyl-ammonium, le bicarbonate de 2-méthoxyéthyl-ammonium, le 2-cyanoéthylcarbonate de 2-cyanoéthyl-ammonium, le bicarbonate de 2-cyanoéthyl-ammonium, l'octadécylcarbonate d'octadécyl-ammonium, le dibutylcarbonate de dibutyl-ammonium, le dioctadécylcarbonate de dioctadécyl-ammonium, le bicarbonate de dioctadécyl-ammonium, le méthyldécylcarbonate de méthyldécyl-ammonium, l'hexaméthylèneiminecarbonate d'hexaméthylèneiminium, le morpholinecarbonate de morpholinium, le benzylcarbonate de benzyl-ammonium, le triéthoxysilylpropylcarbonate de triéthoxysilylpropyl-ammonium, le bicarbonate de pyridinium, l'isopropylcarbonate de triéthylène-diaminium et le bicarbonate de triéthylène-diaminium.

2. Complexe d'argent selon la revendication 1, qui est représenté par la formule 1 ci-dessous :
Ag[A]ₘ (1)
dans laquelle A est un composé de carbamate d'ammonium ou un composé de carbonate d'ammonium tel que défini dans la revendication 1 et 0,7 ≤ m ≤ 2,5.

3. Complexe d'argent selon la revendication 1, dans lequel le composé d'argent représenté par la formule 2 est l'un au moins choisi parmi l'oxyde d'argent, le thiocyanate, le cyanure d'argent, le cyanate d'argent, le carbonate d'argent, le nitrate d'argent, le nitrite d'argent, le sulfate d'argent, le phosphate d'argent, le perchlorate d'argent, le tétrafluoroborate d'argent, l'acétylacétonate d'argent, l'acétate d'argent, le lactate d'argent et l'oxalate d'argent.

4. Complexe d'argent selon la revendication 1, dans lequel le composé d'argent représenté par la formule 2 est l'oxyde d'argent, le carbonate d'argent ou un mélange de ceux-ci.

5. Complexe d'argent selon la revendication 1, qui est obtenu par réaction
- d'oxyde d'argent avec du 2-éthylhexylcarbamate de 2-éthylhexyl-ammonium,
- d'oxyde d'argent avec de l'isopropylcarbamate de isopropyl-ammonium,
- d'oxyde d'argent avec du n-butylcarbamate de n-butyl-ammonium,
- d'oxyde d'argent avec de l'isobutylcarbamate de isobutyl-ammonium,
- d'oxyde d'argent avec du t-butylcarbamate de t-butyl-ammonium,
- de carbonate d'argent avec du 2-éthylhexylcarbamate de 2-éthylhexyl-ammonium,
- d'oxyde d'argent avec du 2-méthoxyéthylcarbamate de 2-méthoxyéthyl-ammonium,
- d'oxyde d'argent avec du 2-cyanoéthylcarbamate de 2-cyanoéthyl-ammonium,
- d'oxyde d'argent avec du morpholinecarbamate de morpholinium,
- d'oxyde d'argent avec de l'hexaméthylèneiminecarbamate d'hexaméthylène-iminium,
- de sulfate d'argent avec du 2-éthylhexylcarbamate de 2-éthylhexyl-ammonium,
- de nitrate d'argent avec du 2-éthylhexylcarbamate de 2-éthylhexyl-ammonium,
- de cyanure d'argent avec du 2-éthylhexylcarbamate de 2-éthylhexyl-ammonium,
- d'oxyde d'argent avec du 2-éthylhexylcarbonate de 2-éthylhexyl-ammonium,
- d'oxyde d'argent avec du bicarbonate de 2-éthylhexyl-ammonium,
- d'oxyde d'argent avec de l'isopropylcarbonate d'isopropyl-ammonium,
- de carbonate d'argent avec de l'isopropylcarbonate d'isopropyl-ammonium,
- de carbonate d'argent avec du 2-éthylhexylcarbonate de 2-éthylhexyl-ammonium,
- d'oxyde d'argent avec du bicarbonate d'isopropyl-ammonium,
- d'oxyde d'argent avec du bicarbonate de 2-méthoxyéthyl-aanmonium,
- de carbonate d'argent avec du bicarbonate de 2-méthoxyéthyl-ammonium,
- de sulfate d'argent avec du 2-éthylhexylcarbonate de 2-éthylhexyl-ammonium,
- de nitrate d'argent avec du 2-éthylhexylcarbonate de 2-éthylhexyl-ammonium, ou
- de cyanure d'argent avec du 2-éthylhexylcarbonate de 2-éthylhexyl-ammonium.

6. Procédé de formation d'un film métallique ou d'un film d'oxyde métallique par formation d'un film mince, à l'aide du complexe d'argent selon l'une quelconque des revendications 1 à 4 et par réalisation d'une oxydation, d'une réduction, d'un traitement thermique, d'un dépôt chimique en phase vapeur, d'un dépôt de plasma en phase vapeur, d'une pulvérisation cathodique, d'un électroplacage, d'une lithographie, d'un traitement IR, avec un faisceau d'électrons ou au laser.

7. Procédé selon la revendication 6, dans lequel le film mince est formé par revêtement sur un substrat.

8. Procédé selon la revendication 7, dans lequel le substrat est choisi parmi le verre, le silicium, le polyester, le polyimide et le papier.

9. Procédé selon la revendication 6, dans lequel le traitement thermique est réalisé à l'aide d'air, d'azote, d'argon, d'hydrogène ou d'un mélange gazeux de ceux-ci.

10. Procédé selon la revendication 7, dans lequel le revêtement est réalisé par revêtement centrifuge, revêtement au rouleau, revêtement par aspersion, revêtement au trempé ou revêtement par écoulement.

11. Procédé selon la revendication 7, dans lequel le revêtement est réalisé par un procédé d'impression choisi parmi l'impression par jet d'encre, l'impression offset, la sérigraphie, l'impression par gravure, et la flexographie.

12. Procédé selon la revendication 7, dans lequel le revêtement est réalisé à l'aide d'une solution de complexe d'argent préparée par dissolution du complexe d'argent dans un solvant choisi parmi un alcool, un glycol, un acétate, un éther, une cétone, un hydrocarbure aliphatique, un hydrocarbure aromatique et un hydrocarbure halogéné.

13. Procédé selon la revendication 12, dans lequel le solvant est l'un au moins choisi parmi le méthanol, l'éthanol, l'isopropanol, le butanol, l'éthylène glycol, la glycérine, l'acétate d'éthyle, l'acétate de butyle, l'acétate de carbitol, l'éther diéthylique, le tétrahydrofurane, le dioxane, la méthyléthylcétone, l'acétone, l'hexane, l'heptane, le benzène, le toluène, le chloroforme, le chlorure de méthylène et le tétrachlorure de carbone.

14. Solution de complexe d'argent préparée par dissolution du complexe d'argent selon l'une quelconque des revendications 1 à 4 dans un solvant choisi parmi un alcool, un glycol, un acétate, un éther, une cétone, un hydrocarbure aliphatique, un hydrocarbure aromatique et un hydrocarbure halogéné.

15. Solution de complexe d'argent selon la revendication 14, dans laquelle le solvant est l'un au moins choisi parmi le méthanol, l'éthanol, l'isopropanol, le butanol, l'éthylène glycol, la glycérine, l'acétate d'éthyle, l'acétate de butyle, l'acétate de carbitol, l'éther diéthylique, le tétrahydrofurane, le dioxane, la méthyléthylcétone, l'acétone, l'hexane, l'heptane, le benzène, le toluène, le chloroforme, le chlorure de méthylène et le tétrachlorure de carbone.

16. Procédé de préparation d'un complexe d'argent par réaction d'au moins un composé d'argent représenté par la formule 2 ci-dessous à la température ambiante en présence d'un solvant :
AgₙX (2)
dans laquelle n est un nombre entier de 1 à 4 et X est un substituant choisi dans le groupe constitué de l'oxygène, du soufre, d'un halogène, d'un groupe cyano, cyanate, carbonate, nitrate, nitrite, sulfate, phosphate, thiocyanate, chlorate, perchlorate, tétrafluoroborate, acétylacétonate et carboxylate ;
avec au moins un composé de carbamate d'ammonium choisi parmi l'éthylcarbamate d'éthyl-ammonium, l'isopropylcarbamate d'isopropyl-ammonium, le n-butylcarbamate de n-butyl-ammonium, l'isobutylcarbamate d'isobutyl-ammonium, le t-butylcarbamate de t-butyl-ammonium, le 2-éthylhexylcarbamate de 2-éthylhexyl-ammonium, l'octadécylcarbamate d'octadécyl-ammonium, le 2-méthoxyéthylcarbamate de 2-méthoxyéthyl-ammonium, le 2-cyanoéthylcarbamate de 2-cyanoéthyl-ammonium, le dibutylcarbamate de dibutyl-ammonium, le dioctadécylcarbamate de dioctadécyl-ammonium, le méthyldécylcarbamate de méthyldécyl-ammonium, l'hexaméthylèneiminecarbamate d'hexaméthylèneiminium, le morpholinecarbamate de morpholinium, l'éthylhexylcarbamate de pyridinium, l'isopropylbicarbamate de triéthylènediaminium, le benzylcarbamate de benzyl-ammonium et le triéthoxysilylpropylcarbamate de triéthoxysilylpropyl-ammonium ; et/ou
au moins un composé de carbonate d'ammonium choisi parmi l'éthylcarbonate d'éthyl-ammonium, l'isopropylcarbonate d'isopropyl-ammonium, le bicarbonate d'isopropyl-ammonium, le n-butylcarbonate de n-butyl-ammonium, l'isobutylcarbonate d'isobutylammonium, le t-butylcarbonate de t-butyl-ammonium, le bicarbonate de t-butyl-ammonium, le 2-éthylhexylcarbonate de 2-éthylhexyl-ammonium, le bicarbonate de 2-éthylhexyl-ammonium, le 2-méthoxyéthylcarbonate de 2-méthoxyéthyl-ammonium, le bicarbonate de 2-méthoxyéthyl-ammonium, le 2-cyanoéthylcarbonate de 2-cyanoéthyl-ammonium, le bicarbonate de 2-cyanoéthyl-ammonium, l'octadécylcarbonate d'octadécyl-ammonium, le dibutylcarbonate de dibutyl-ammonium, le dioctadécylcarbonate de dioctadécyl-ammonium, le bicarbonate de dioctadécyl-ammonium, le méthyldécylcarbonate de méthyldécyl-ammonium, l'hexaméthylèneiminecarbonate d'hexaméthylèneiminium, le morpholinecarbonate de morpholinium, le benzylcarbonate de benzyl-ammonium, le triéthoxysilylpropylcarbonate de triéthoxysilylpropyl-ammonium, le bicarbonate de pyridinium, l'isopropylcarbonate de triéthylène-diaminium et le bicarbonate de triéthylène-diaminium.

17. Procédé selon la revendication 16, dans lequel le complexe d'argent est représenté par la formule 1 ci-dessous :
Ag[A]ₘ (1)
dans laquelle A est un composé de carbamate d'ammonium ou un composé de carbonate d'ammonium tel que défini dans la revendication 16 et 0,7 ≤ m ≤ 2,5.

18. Procédé selon la revendication 16, dans lequel le solvant est l'un au moins choisi parmi le méthanol, l'éthanol, l'isopropanol, le butanol, l'éthylène glycol, la glycérine, l'acétate d'éthyle, l'acétate de butyle, l'acétate de carbitol, l'éther diéthylique, le tétrahydrofurane, le dioxane, la méthyléthylcétone, l'acétone, l'hexane, l'heptane, le benzène, le toluène, le chloroforme, le chlorure de méthylène et le tétrachlorure de carbone.

19. Complexe d'argent qui est obtenu par réaction
- d'oxyde d'argent avec du n-propylcarbamate de n-propyl-ammonium,
- d'oxyde d'argent avec du 2-hydroxyéthylcarbamate de 2-hydroxyéthyl-ammonium,
- d'oxyde d'argent avec de l'aminoéthylcarbamate d'aminoéthyl-ammonium,
- d'oxyde d'argent avec du 2-éthylhexylcarbamate de 2-éthylhexyl-ammonium et de l'aminoéthylcarbamate d'aminoéthyl-ammonium,
- de carbonate d'argent avec du bicarbonate d'octyl-ammonium,
- d'oxyde d'argent avec du bicarbonate d'isobutylammonium,
- d'oxyde d'argent avec du bicarbonate de n-butyl-ammonium,
- d'oxyde d'argent avec du bicarbonate de morpholinium,
- d'oxyde d'argent avec de l'aminoéthylcarbonate d'aminoéthyl-ammonium,
- d'oxyde d'argent avec du 2-éthylhexylcarbonate de 2-éthylhexyl-ammonium et de l'aminoéthylcarbonate d'aminoéthyl-ammonium,
- d'oxyde d'argent avec du carbamate d'ammonium,
- de carbonate d'argent avec du carbamate d'ammonium,
- d'oxyde d'argent avec du carbonate d'ammonium,
- de carbonate d'argent avec du carbonate d'ammonium,
- d'oxyde d'argent avec du bicarbonate d'ammonium, ou
- de carbonate d'argent avec du bicarbonate d'ammonium.
